(19) **Europäisches Patentamt / European Patent Office / Office européen des brevets**

(11) **EP 4 076 314 B1**

(12) **EUROPÄISCHE PATENTSCHRIFT**

(45) Veröffentlichungstag und Bekanntmachung des Hinweises auf die Patenterteilung:
**29.01.2025 Patentblatt 2025/05**

(21) Anmeldenummer: **20841898.8**

(22) Anmeldetag: **17.12.2020**

(51) Internationale Patentklassifikation (IPC):
**A61F 13/0206** (2024.01)   **A61F 13/0203** (2024.01)
**A61F 13/06** (2006.01)   **A61F 13/10** (2006.01)

(52) Gemeinsame Patentklassifikation (CPC):
**A61F 13/0206; A61F 13/0209; A61F 13/0226; A61F 13/069; A61F 13/101**

(86) Internationale Anmeldenummer:
**PCT/EP2020/086836**

(87) Internationale Veröffentlichungsnummer:
**WO 2021/123028 (24.06.2021 Gazette 2021/25)**

(54) **MEDIZINISCHER ARTIKEL ZUR PROPHYLAXE VON DEKUBITALULCERA**

MEDICAL ARTICLE FOR THE PROPHYLAXIS OF DECUBITUS ULCERS

ARTICLE MÉDICAL POUR LA PRÉVENTION DES ESCARRES DE DÉCUBITUS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.12.2019 DE 102019135061**

(43) Veröffentlichungstag der Anmeldung:
**26.10.2022 Patentblatt 2022/43**

(73) Patentinhaber: **Paul Hartmann Aktiengesellschaft 89522 Heidenheim (DE)**

(72) Erfinder:
• **ECKSTEIN, Axel 89522 Heidenheim (DE)**
• **KRÄMER, Maja 89542 Herbrechtingen (DE)**

(74) Vertreter: **Paul Hartmann AG Patents & Licensing Paul-Hartmann-Straße 12 89522 Heidenheim (DE)**

(56) Entgegenhaltungen:
EP-A1- 3 085 344   EP-A1- 3 085 344
EP-A1- 3 498 243   EP-A1- 3 498 243
EP-B1- 3 085 344   WO-A1-2014/058532
WO-A1-2014/058532   DE-A1- 102007 049 428
DE-A1- 102007 049 428   US-A1- 2018 344 533
US-A1- 2018 344 533

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft medizinische Artikel zur Prophylaxe der Entstehung und/oder Verschlechterung von Dekubitalulcera.

Der Dekubitalulcus (Synonyme: Dekubitus, Dekubitalgeschwür, Druckgeschwür, Wundliegegeschwür oder jeweils -ulkus) ist definiert als durch äußere (längerfristige) Druckeinwirkung mit Kompression von Gefäßen und lokaler Ischämie hervorgerufene trophische Störung von Geweben (v. a. Haut und Unterhautgewebe) mit Nekrose, Mazeration, evtl. Infektion. Dekubitalulcera entstehen vor allem bei Bettlägerigkeit, insbesondere an Körperstellen, an denen die Haut einem Knochen unmittelbar anliegt, aber auch z.B. unter schlechtsitzenden Prothesen und zu engen Gipsverbänden.

Der Dekubitus wird in folgende Stadien eingeteilt. Hierbei sind Dekubitalulcera der Stufe II, Stufe III und Stufe IV als chronische Wunden bekannt:

Stufe I: Hierbei handelt es sich um eine persistierende, umschriebene Hautrötung, die auch bei Entlastung bestehen bleibt. Die Rötung ist scharf umgrenzt und kann verhärten oder überwärmt sein. Die Haut ist noch intakt.

Stufe II: In dieser Phase kommt es zu Blasenbildung und Hautabschürfung und damit zu Teilverlust der Haut. Die Epidermis bis hin zu Anteilen der Dermis ist geschädigt. In dieser Phase liegt eine oberflächige Wunde oder ein flaches Geschwür vor.

Stufe III: In diesem fortgeschrittenen Stadium ist bereits ein Verlust aller Hautschichten zu beobachten. Darüber hinaus sind eine Schädigung der subkutanen Gewebe und eventuell Nekrosen zu beobachten, die bis auf das darunter liegende Muskelgewebe reichen können. Erfahrungsgemäß muss es erst zu einer Abgrenzung des nekrotischen Gewebes kommen bis das ganze Ausmaß des Gewebeschadens erkennbar wird. Der Dekubitus III zeigt sich klinisch als offenes, tiefes Geschwür.

Stufe IV: In diesem äußerst kritischen Stadium ist ein Verlust aller Hautschichten mit ausgedehnter Zerstörung, Gewebsnekrose oder Schädigung von Muskeln, Knochen oder unterstützenden Strukturen (Sehnen, Gelenkkapseln) zu verzeichnen. Der Dekubitus IV zeigt sich klinisch als großflächiges, offenes und tiefes Geschwür.

Dekubitalulcera können entstehen, wenn Druck auf weiches Gewebe ausgeübt wird, so dass der Blutfluss in dieses Gewebe vollständig oder teilweise unterbrochen wird. Scherkräfte, die durch Reibung auf bestimmte Hautstellen entstehen, tragen ebenfalls in bedeutendem Maße zur Entstehung von Dekubitalulcera bei. Derartige Scherkräfte entstehen durch Bewegung der Haut auf Oberflächen anderer Materialien, wie beispielsweise einem Bettlaken. Wenn sich aufgrund der auftretenden Scherkräfte die Haut eines Patienten gegen die unter der Haut liegenden Gewebe verschiebt, werden in diesen Geweben befindliche Blutgefäße ebenfalls zusammengedrückt, so dass der Blutfluss in diesen Geweben eingeschränkt oder unterbrochen wird. Bevorzugt entstehen Dekubitalulcera an bestimmten Hautstellen, beispielsweise an der Haut, die die Sakralregion, das Steißbein, die Ferse oder die Hüftknochen bedeckt. Weitere Stellen, an denen sie häufig auftreten sind Ellbogen, Knie, Gelenke, Schultern. Meistens entstehen Dekubitalulcera bei Patienten, deren Bewegung stark beeinträchtigt oder vollständig unterbunden wird, wie beispielsweise bei bettlägerigen Patienten oder solchen, die größtenteils auf einen Rollstuhl angewiesen sind. Bei diesen Patienten ist das Vermögen eingeschränkt, durch eigenständige Bewegung der Gliedmaßen, an denen sich unter Druck stehende Partien befinden, eine Druckentlastung herbeizuführen. Andere Faktoren können eine wichtige Rolle bei der Entstehung von Dekubitalulcera spielen, beispielsweise ein Proteinmangel, ungünstiges Mikroklima (Hautfeuchtigkeit infolge von Schwitzen, Wundexsudat oder Inkontinenz), Gefäßerkrankungen wie Arteriosklerose oder Erkrankungen, die die Sensibilität der Haut einschränken wie Paralyse oder Neuropathie. Die Heilungsrate von Dekubitalulcera kann stark eingeschränkt sein aufgrund des Patientenalters, des medizinischen Zustands des Patienten, Rauchen oder Medikamente. Häufig ist es schwierig, bei anfälligen Patienten die Entstehung von Dekubitalulcera erfolgreich zu verhindern beziehungsweise der Verschlechterung bereits bestehender Dekubitalulcera vorzubeugen. Zu den üblichen Maßnahmen gehören eine Druckverteilung durch häufige Lagenwechsel der Patienten oder die Ausstattung des Liegeplatzes oder eines Rollstuhls mit druckmindernden Matratzen oder Kissen. Medizinische Verbände zur Vorbeugung von Dekubitalulcera sind zum Beispiel aus EP 3 498 243 A1 bekannt. Die Mechanismen, die zur Entstehung von Dekubitalulcera führen, sind nicht vollständig verstanden. Da auf Haut und Gewebe wirkender Druck und Scherkräfte die Entstehung von Dekubitalulcera begünstigen, sollten Maßnahmen, die der Prophylaxe der Entstehung oder der Verschlechterung von Dekubitalulcera dienen, darauf ausgerichtet sein, diese Größen zu verringern.

Es besteht daher ein Bedürfnis, Erzeugnisse zur Verfügung zu stellen, mit deren Anwendung der Entstehung von Dekubitalulcera wirksam vorgebeugt werden kann. Weiterhin besteht ein Bedürfnis, Erzeugnisse zur Verfügung zu stellen, mit deren Anwendung die Verschlechterung bereits entstandener Dekubitalulcera verhindert werden kann.

Die vorliegende Erfindung gemäß Anspruch 1 löst die vorgenannten Probleme.

In der vorliegenden Anmeldung wird unter dem Begriff "proximale Oberfläche" die Seite einer Schicht oder einer Lage verstanden, die der durch den Artikel abgedeckten Haut zugewandt ist, während der Begriff "distale Oberfläche" die Seite bezeichnet, die der Haut abgewandt ist. Sofern im folgenden beschriebene Bestandteile erfindungsgemäßer medizinischer Artikel eine im weitesten Sinne flächenhafte Ausdehnung aufweisen, besitzen derartige Bestandteile jeweils eine proximale und eine distale Oberfläche. Diese Flächen werden an ihren Seiten begrenzt. Sofern im Rahmen der vorliegenden Anmeldung der Begriff "allseitig" verwendet wird, soll dieser so verstanden werden, dass alle eine Oberfläche einer Lage oder Schicht begrenzenden Seiten damit gemeint sind.

Ein medizinischer Artikel gemäß der vorliegenden Erfindung umfasst eine Kompresse und eine an zu behandelnder Haut adhäsive Hautkontaktschicht. Die Kompresse umfasst einen Kern und eine den Kern umgebende Hülle. Der Kern umfasst ein Vliesmaterial aus Fasern und absorbierenden Partikeln. Die Kompresse weist eine Längsrichtung mit einem ersten Elastizitätsmodul und eine Querrichtung mit einem zweiten Elastizitätsmodul auf. Dabei ist der erste Elastizitätsmodul größer als der zweite Elastizitätsmodul.

Die Hülle weist eine erste Lage aus einem flüssigkeitsdurchlässigen Material auf, die auf der proximalen Oberfläche des Kerns angeordnet ist und eine zweite Lage aus einem von dem Material der ersten Lage der Hülle verschiedenen Material, die auf der distalen Oberfläche des Kerns angeordnet ist. Dabei überragt die erste Lage der Hülle allseitig die proximale Oberfläche des Kerns und die zweite Lage der Hülle allseitig die distale Oberfläche des Kerns, so dass die erste Lage der Hülle und die zweite Lage der Hülle jeweils einen Randbereich bilden, der den Kern allseitig umgibt. Die erste Lage der Hülle und die zweite Lage der Hülle sind in diesem Randbereich miteinander verbunden.

Zur Klarstellung sei erwähnt, dass die Begriffe Längsrichtung und Querrichtung nicht in dem Sinne verstanden werden sollen, dass ein erfindungsgemäßer medizinischer Artikel in einer der beiden Richtungen eine größere Längenausdehnung aufweist als in der anderen Richtung. Erfindungsgemäße medizinische Artikel können auch eine im Wesentlichen quadratische oder kreisförmige Form aufweisen.

Unter Anisotropie einer Schicht oder Lage wird im Rahmen der vorliegenden Erfindung verstanden, dass ihre mechanischen Eigenschaften in Abhängigkeit von der Messrichtung unterschiedlich ausgeprägt sind. Bevorzugt weist eine anisotrope Schicht oder Lage im Sinne der vorliegenden Erfindung eine Anisotropie hinsichtlich ihres Elastizitätsmoduls auf. Bevorzugt weist eine Schicht oder Lage eine Längsrichtung mit einem ersten Elastizitätsmodul und eine zur Längsrichtung rechtwinklige Querrichtung mit einem zweiten Elastizitätsmodul auf, wobei der erste Elastizitätsmodul größer ist als der zweite Elastizitätsmodul.

Bevorzugt korrespondiert die Längsrichtung der Kompresse mit der Richtung, die rechtwinklig zur Maschinenrichtung des Herstellungsverfahrens des medizinischen Artikels ist, während die Querrichtung mit der Richtung korrespondiert, die parallel zur Maschinenrichtung des Herstellungsverfahrens des medizinischen Artikels ist.

Der Kern weist eine proximale und eine distale Oberfläche auf. Der Kern umfasst ein Vliesmaterial aus Fasern und absorbierenden Partikeln. Das Material kann jede Art von Wundexsudat-absorbierendem Material enthalten, insbesondere absorbierende Fasern und absorbierende Partikel. Ein derartiges Material erfüllt im erfindungsgemäßen Artikel eine Doppelfunktion, da es aufgrund der Fasern enthaltenden Struktur eine vorteilhafte Polsterwirkung entfalten kann und aufgrund der absorbierenden Partikel einen vorteilhaften Beitrag zur Verbesserung des Mikroklimas an der betroffenen Hautstelle leistet. So kann Feuchtigkeit, die beispielsweise aufgrund von Hautatmung, Schweißbildung oder dem Austritt von Wundexsudat auftritt, durch die Partikel aufgenommen werden. Wie vorgehend beschrieben begünstigt das Auftreten von Flüssigkeit an der Hautoberfläche eine Erhöhung der Scherkräfte zwischen Hautoberfläche und einem an der Haut anliegenden Material, wodurch die Entstehung neuer Dekubitalulcera beziehungsweise die Verschlechterung bereits bestehender Dekubitalulcera gefördert werden kann. Ein erfindungsgemäßer Artikel wirkt aufgrund der Flüssigkeitsaufnahme diesem Risiko entgegen.

Geeigneter Weise enthält das Material eine Mischung aus absorbierenden Fasern und absorbierenden Partikeln. Dabei erfüllt eine Vlieslage aus Fasern und absorbierenden Partikeln in besonderer Weise die genannte Doppelfunktion, wenn die absorbierenden Partikel im Wesentlichen gleichmäßig im Vliesmaterial aus Fasern verteilt sind. Bei der Aufnahme von Flüssigkeit quellen die absorbierenden Partikel und bilden gelartige Strukturen, die mit den umgebenden Fasern eine Verbindung eingehen und so einen netzartigen Verbund aus Fasern und Gelpartikeln bilden, die besonders geeignet sind, Druck und Scherkräfte von der Hautoberfläche und dem darunter liegenden Gewebe abzulenken.

Geeignete Materialien für absorbierende Fasern können beispielsweise Cellulose oder Cellulose-basierte Polymere, Viskose, Polyester, Polyamid sowie Derivate, Copolymere und Mischungen davon sein. Darüber hinaus sind Fasern geeignet, die superabsorbierende Eigenschaften aufweisen und auf Polyacrylsäure, Natriumpolyacrylat, Polyacrylsäureestern, basieren sowie auf deren Derivaten, Copolymeren und Mischungen. Ein bevorzugtes Material ist Cellulose.

Geeignete Materialien für absorbierende Partikel beinhalten Cellulosederivate, Alginate, Carboxymethylcellulose und deren Derivate, Copolymere, Mischungen und Salze, sowie superabsorbierende Partikel umfassend Polyacrylsäure, Natriumpolyacrylate, Polyacrylsäureester sowie Derivate, Copolymere und Mischungen davon.

In einer bevorzugten Ausführungsform umfasst der Kern der Kompresse eine Mischung absorbierender Fasern und superabsorbierender Materialien. Absorbierende Fasern bewirken eine schnelle Aufnahme von Wundexsudat, während superabsorbierende Materialien eine hohe Absorptionskapazität bewirken. Geeignete absorbierende Fasern können Cellulose-basierte Materialien sein. In einer bevorzugten Ausführungsform bestehen die absorbierenden Fasern im Wesentlichen aus Cellulosefasern. Geeignete superabsorbierende Materialien liegen in Form von Partikeln vor.

In einer bevorzugten Ausführungsform enthält der Kern der Kompresse eine Mischung aus absorbierenden Fasern, die im Wesentlichen aus Cellulose bestehen, und superabsorbierenden Partikeln, die Polyacrylsäure und Natriumpolyacrylat enthalten. Ein derartiger Kern bewirkt eine rasche Aufnahme von Feuchtigkeit, insbesondere Wundexsudat sowie eine schnelle Verteilung der Feuchtigkeit innerhalb des Kerns.

Das superabsorbierende Material kann in einer Menge von 1 Gew.-% bis zu 99 Gew.-% innerhalb des Kerns der Kompresse enthalten sein. Bevorzugt stellt die Menge des superabsorbierenden Materials einen Anteil von 30 Gew.-% bis 55 Gew.-% des Kerns der Kompresse dar.

Grundsätzlich wird unter dem Begriff "superabsorbierendes Material" ein wasserunlösliches, quellbares Polymer verstanden, das in der Lage ist, ein Vielfaches seines Eigengewichts an Flüssigkeit wie Wasser, Salzlösung oder Körperflüssigkeiten, z.B. Wundexsudat aufzunehmen und zu binden. Die Flüssigkeitsabsorption resultiert in der Bildung eines Hydrogels. Die Absorptionskapazität für reines Wasser ist in der Regel größer als für eine Salzlösung oder Körperflüssigkeit. Im Zusammenhang mit der vorliegenden Erfindung wird unter dem Begriff "superabsorbierendes Material" ein Material verstanden, das einen w-Wert für die freie Quellkapazität entsprechend der Standardmessmethode WSP 240.2 (05) von wenigstens 10 g/g, bevorzugt wenigstens 20 g/g aufweist. Die Messmethode WSP 240.2 (05) zur Bestimmung des w-Wertes ist beschrieben in "Standard Test Methods fort he Nonwovens and Related Industries", Ausgabe 2008 (veröffentlicht durch "EDANA, International Association Serving the Nonwovens and Related Industries", Cary, N.C., USA und "INDA, Association oft he Nonwoven Fabric Industry", Brüssel, Belgien).

In einer bevorzugten Ausführungsform weist der Kern der Kompresse eine Absorptionskapazität von 100 g/ 100 cm$^2$ bis 5000 g/ 100 cm$^2$, bevorzugt von 120 g/ 100 cm$^2$ bis 4000 g/ 100 cm$^2$, besonders bevorzugt von mehr als 130 g/ 100 cm$^2$ bis 3000 g/ 100 cm$^2$ gemäß einer Testmethode, die in Beispiel 4 der vorliegenden Anmeldung beschrieben wird.

Der Kern wird von einer Hülle umgeben. Unter einer Hülle ist ein Element zu verstehen, welches den Kern vollständig umgibt. Die Hülle kann aus einer oder mehreren Lagen gebildet werden. Dabei können einzelne Lagen die Oberflächen des Kerns überragen. Die Lagenteile, die den Kern überragen, können flächenhaft orientiert sein und so einen den Kern überragenden Randbereich bilden. Alternativ können die Lagenteile, die den Kern überragen, im überragenden Bereich um eine Kante des Kernmaterials umgeschlagen werden, so dass das Kernmaterial von diesem Materialabschnitt umwickelt wird.

Erfindungsgemäß umfasst die Hülle eine erste Lage aus einem flüssigkeitsdurchlässigen Material, welches hydrophile Eigenschaften besitzt. Das Material kann ein Material umfassen, das hydrophile Eigenschaften aufgrund seiner chemischen Natur aufweist, oder ein Material, welches aufgrund seiner chemischen Natur originär hydrophobe Eigenschaften aufweist und in einem chemischen oder physikalischen Verfahren so behandelt wurde, dass seine Oberfläche hydrophile Eigenschaften aufweist. Geeignete Verfahren zur Hydrophilisierung originär hydrophober Materialien umfassen elektrochemische Prozesse, Flammenbehandlung, Coronabehandlung und Plasmabehandlung.

Geeignete Materialien für die erste Lage der Hülle sind Cellulose, Polyester, Polyamid, Polyethylen, Polypropylen sowie Copolymere aus zwei oder mehr der vorgenannten Materialien. Bevorzugt umfasst die erste Lage eines hydrophilen Materials entweder ein Polypropylenvlies, das physikalisch so behandelt wurde, dass seine Oberfläche hydrophile Eigenschaften zeigt, oder ein Vliesmaterial, das 55 % Polyamidfasern und 45 % Viskosefasern mit einem Flächengewicht von 37 g/m$^2$ enthält. Ein derartiges Material kann unter der Bezeichnung M1526 von der Firma Freudenberg (Deutschland) bezogen werden. Alternativ bevorzugt ist ein Vlies umfassend 63 % Polypropylenfasern und 37 % Viskosefasern mit einem Flächengewicht von 45 g/m$^2$, welches unter der Bezeichnung 670532/2 von der Firma Freudenberg (Deutschland) bezogen werden kann.

Erfindungsgemäß umfasst die Hülle eine zweite Lage aus einem Material, welches hydrophobe Eigenschaften aufweist. Das Material kann hydrophobe Eigenschaften aufgrund seiner chemischen Natur aufweisen oder ein Material enthalten, welches originär hydrophile Eigenschaften aufweist, das aber durch chemische oder physikalische Behandlung so verändert wurde, dass es hydrophobe Eigenschaften zeigt, beispielsweise durch eine Behandlung mit Fluorkohlenwasserstoffen, Silikonen, Alkanen etc. Geeignete Grundmaterialien für die zweite Lage der Hülle sind Cellulose, Polyester, Polyamid, Polyethylen, Polypropylen sowie Copolymere aus zwei oder mehr der vorgenannten Materialien.

Die erste Lage der Hülle umfasst ein erstes Material mit hydrophilen Eigenschaften und die zweite Lage der Hülle ein zweites Material mit hydrophoben Eigenschaften.

In einer weiteren bevorzugten Ausführungsform umfasst die zweite Lage der Hülle ein im Wesentlichen flüssigkeitsundurchlässiges Material.

Beide Lagen der den Kern allseitig umgebenden Hülle können in beliebiger Farbe gestaltet sein. Üblicherweise haben medizinische Artikel eine weiße Farbe, was Reinheit und Sterilität signalisiert. In einer weiteren Ausführungsform weist wenigstens eine der beiden Lagen eine hellgrüne Farbe auf. Diese Farbe bewirkt einen starken Farbkontrast, wenn die Lage mit Flüssigkeit befeuchtet ist, so dass eine Pflegekraft unmittelbar durch äußerliche Betrachtung eines am Patienten angelegten medizinischen Artikels erkennen kann, wenn der Kern Flüssigkeit enthält oder wenn das maximale Aufnahmevermögen des Artikels erreicht worden ist. Das Vorhandensein oder die Menge absorbierter Flüssigkeit innerhalb des Kerns eines erfindungsgemäßen Artikels kann einen deutlichen Hinweis darauf geben, dass im Bereich der von dem Artikel abgedeckten Haut ein Dekubitalulcus gerade entstanden ist oder dass sich der Grad eines durch den Artikel abgedeckten Dekubitalulcus verändert hat. Überraschenderweise wurde festgestellt, dass eine grüne Farbe entsprechend RAL6019 einen besonders vorteilhaften Farbkontrast mit Wundexsudat gewährleistet und dabei weiterhin von Patienten und Pflegepersonal als rein und steril wahrgenommen wird.

Die erste und die zweite Lage der Hülle des Kerns sind miteinander verbunden. Diese Verbindung kann durch jeden geeigneten Prozess zur Verbindung von Materialien erreicht werden, sei es unmittelbar oder mittelbar.

In einer bevorzugten Ausführungsform umfassen sowohl die erste Lage als auch die zweite Lage der Hülle jeweils ein Material mit thermoplastischen Eigenschaften. So ist es möglich, die erste und die zweite Lage der Hülle in einem thermischen Verfahrensschritt miteinander zu verbinden. Thermische Verbindungsverfahren können in kontinuierlicher Weise durchgeführt und somit kostengünstiger als andere Verfahren eingesetzt werden. Geeignete thermische Verfahren zur Verbindung von Materialien beinhalten Hitzeschweißen, Laserschweißen und Ultraschallschweißen.

In einer bevorzugten Ausführungsform umfasst die zweite Lage der Hülle ein thermoplastisches Material mit hydrophoben Eigenschaften und die erste Lage der Hülle ein thermoplastisches Material, welches das gleiche Material wie das der zweiten Lage der Hülle ist und in einem chemischen und/oder physikalischen Verfahren so behandelt wurde, dass es hydrophile Eigenschaften aufweist. Wenn die erste und die zweite Lage Materialien unterschiedlicher chemischer Natur aufweisen, können die physikalischen Eigenschaften signifikant voneinander abweichen. Dies kann sich nachteilig in einem Verbindungsverfahren auswirken, in dem Bedingungen gefunden werden müssen, die kompatibel mit allen beteiligten Materialien sein müssen. In einem thermischen Verbindungsverfahren muss die Betriebstemperatur höher sein als die Schmelzpunkte oder Schmelzbereiche jedes einzelnen beteiligten Materials. Allerdings können thermolabile Materialien bei zu hohen Temperaturen zerstört werden. Daher kann die Auswahl geeigneter Materialien für die erste und die zweite Lage in Abhängigkeit der chemischen Natur der Materialien erheblich beeinträchtig sein. Wenn die erste und die zweite Lage Materialien derselben chemischen Natur umfassen, unterscheiden sich diese Materialien nicht in ihren physikalischen Eigenschaften, die für die Durchführung eines Verbindungsverfahrens relevant sind. Dies ist besonders vorteilhaft bei thermischen Verbindungsverfahren, wenn die Prozesstemperatur nur gering oberhalb der Schmelzbereiche der beteiligten Materialien liegt. So wird das Risiko für eine thermische Zerstörung erheblich reduziert.

In einer bevorzugten Ausführungsform umfasst das thermoplastische Material sowohl der ersten wie auch der zweiten Lage der Hülle Polypropylen.

In einer bevorzugten Ausführungsform wurden die erste Lage und die zweite Lage der den Kern umgebenden Hülle in einem thermischen Verfahren miteinander verbunden und weisen daher eine Schweißverbindung miteinander auf. Eine Schweißverbindung aus einem thermischen Verbindungsverfahren weist eine vorteilhafte Verbindung aufgrund ihrer Schweißfestigkeit und Flexibilität auf. Schweißfestigkeit bedeutet im Rahmen der vorliegenden Erfindung die Kraft, die notwendig ist, um die beiden miteinander verbundenen Lagen zu trennen. Die Schweißfestigkeit kann mit einer Methode bestimmt werden, die in den Beispielen dieser Anmeldung beschrieben ist. In einer bevorzugten Ausführungsform ist die Schweißfestigkeit größer als 0,75 N/15 mm, bevorzugt größer als 1 N /15 mm.

Eine hohe Schweißfestigkeit ist vorteilhaft um eine unbeabsichtigte Delaminierung der miteinander verbundenen Materialien zu verhindern. Delaminierung der ersten und der zweiten Lage der Hülle kann selbst in geringem Ausmaß zu Undichtigkeiten innerhalb des Artikels führen. In diesem Fall können zusätzliche Scherkräfte innerhalb des Artikels auftreten, die die Entstehung beziehungsweise Verschlechterung von Dekubitalulcera weiter begünstigen.

Flexibilität bedeutet im Rahmen der vorliegenden Erfindung die Eigenschaft, die ein Material in die Lage versetzt, seine Konformation zu verändern. Sie wird durch die Steifheit des Materials beschrieben.

Flexibilität ist für einen medizinischen Artikel bedeutend um sich an die Körperoberfläche eines Patienten anzupassen, die häufig unregelmäßig ist. Darüber hinaus sind unflexible Verbände sehr steif und verursachen bei Bewegung Druck und Stress auf die Hautoberfläche eines Patienten, was Schmerz verursachen kann und die Entstehung oder Veschlechterung von Dekubitalulcera begünstigen kann.

In einer bevorzugten Ausführungsform umfasst die Schweißverbindung wenigstens eine diskontinuierliche Schweißlinie. Eine diskontinuierliche Schweißlinie zeigt eine vorteilhafte Flexibilität, welche sich positiv für einen Patienten auf den Tragekomfort auswirkt und der Entstehung oder Verschlechterung von Dekubitalulcera entgegenwirkt.

In einer bevorzugten Ausführungsform umfasst die Schweißverbindung vier bis sechs parallele diskontinuierliche Schweißlinien, was sich in einer vorteilhaften Ausbalancierung der Eigenschaften Schweißfestigkeit und Flexibilität auswirkt.

In einer bevorzugten Ausführung ist die wenigstens eine diskontinuierliche Schweißlinie parallel zur Maschinenrichtung im Herstellungsprozess ausgerichtet.

Die Kompresse kann außerdem eine Diffusionsschicht enthalten. Eine derartige Schicht ermöglicht eine schnellere Verteilung von Feuchtigkeit, insbesondere Wundexsudat in horizontaler Richtung innerhalb des Kerns der Kompresse. Diese Schicht kann sich auf der proximalen Oberfläche des Kerns zwischen der proximalen Lage der Hülle und der proximalen Oberfläche des Kerns befinden. Die Diffusionsschicht kann auch eine Materiallage sein, die den gesamten Kern umhüllt. Bevorzugt ist eine Diffusionsschicht eine Papierschicht, die den Kern komplett umhüllt. In einer bevorzugten Ausführungsform ist dieses Papier aus Cellulose hergestellt und hat ein Flächengewicht von 15 bis 20 g/m$^2$.

Die an zu behandelnder Haut adhäsive Hautkontaktschicht umfasst ein Material, welches einen hautfreundlichen Klebstoff aufweist. Geeignete Klebstoffe können auf Acrylaten, Acrylatestern, Polyvinylethylether, Polyurethan, Silikon, deren Derivaten, Mischungen oder Copolymeren basieren wie beispielsweise in GB1280631 beschrieben. Bevorzugt handelt es sich um einen druckempfindlichen Klebstoff.

In einer bevorzugten Ausführungsform umfasst die an zu behandelnder Haut adhäsive Hautkontaktschicht eine Lage eines hautfreundlichen Silikonklebstoffs. Geeigneter Weise ist der Silikonklebstoff ein sogenanntes weiches Hautklebstoff-Silikonelastomer. Die gesamte Beschichtungsmasse des Silikonklebstoffs beträgt in geeigneter Weise von 15 g/m$^2$ bis 500 g/m$^2$, bevorzugt 50 g/m$^2$ bis 250 g/m$^2$, besonders bevorzugt 100 g/m$^2$ bis 200 g/m$^2$.

Überraschenderweise wurde herausgefunden, dass ein derartiger medizinischer Artikel vorteilhafte Eigenschaften hinsichtlich der Haftkraft auf Haut aufweist. Eine Methode zur Bestimmung der Haftkraft auf Haut wird in den Beispielen der vorliegenden Anmeldung beschrieben. Ein geeigneter medizinischer Artikel muss auf Haut ohne Einbußen der Haftkraft haften können, so dass eine Anwendung des Artikels über mehrere Stunden gewährleistet werden kann, bevorzugt eine Anwendung über wenigstens einen Tag, besonders bevorzugt über wenigstens drei Tage, am meisten bevorzugt eine Anwendung über sieben Tage. Andererseits muss ein Artikel hautfreundliche Eigenschaften aufweisen, die es ermöglichen, den Artikel nach erfolgter Anwendung zu entfernen ohne beim Patienten Schmerz oder Hautirritationen zu verursachen. Überraschenderweise wurde herausgefunden, dass ein Artikel mit einer Haftkraft von 200 mN/ cm, bevorzugt 350 mN/ cm und 650 mN/ cm in dieser Hinsicht vorteilhaft ist.

In einer bevorzugten Ausführungsform umfasst die an zu behandelnder Haut adhäsive Hautkontaktschicht Öffnungen, die eine Passage von Wundexsudat zum Kern ermöglichen. Diese Öffnungen können jede geometrische Form aufweisen, regelmäßig oder unregelmäßig, insbesondere eine kreisförmige, ovale, dreieckige, quadratische, fünfeckige, sechseckige oder andere polygonale Form mit einheitlicher oder uneinheitlicher Seitenlänge.

In einer bevorzugten Ausführungsform umfasst die an zu behandelnder Haut adhäsive Hautkontaktschicht Öffnungen mit beliebiger Form, die eine durchschnittliche offene Fläche von 0,03 mm$^2$ bis zu 7,0 mm$^2$ aufweisen.

In einer bevorzugten Ausführungsform weist die an zu behandelnder Haut adhäsive Hautkontaktschicht Öffnungen auf, deren gesamte offene Fläche zwischen 10 % und 25 % der gesamten Oberfläche der Hautkontaktschicht beträgt. Überraschenderweise wurde herausgefunden, dass eine offene Fläche in diesem Bereich in einer bevorzugten Absorptionsgeschwindigkeit von Wundexsudat resultiert. Im Rahmen der vorliegenden Erfindung bedeutet Absorptionsgeschwindigkeit die Zeit, die zur Absorption von Wundexsudat benötigt wird. Sie kann durch eine Testmethode bestimmt werden, die in den Beispielen der vorliegenden Anmeldung beschrieben ist. Überraschenderweise wurde herausgefunden, dass die Absorptionsgeschwindigkeit besonders vorteilhaft ist, wenn die an zu behandelnder Haut adhäsive Hautkontaktschicht eine offene Fläche von 11 % bis 22 %, bevorzugt 12 % bis 18 %. Geeigneter Weise ist die Dichte der Öffnungen zwischen 1000 bis 1000000 Öffnungen pro Quadratmeter, beispielsweise 5000 bis 50000 Öffnungen pro Quadratmeter.

In einer bevorzugten Ausführungsform umfasst die an zu behandelnder Haut adhäsive Hautkontaktschicht Öffnungen mit einer im Wesentlichen kreisförmigen Form, deren mittlerer Durchmesser zwischen 0,2 mm bis zu 3,0 mm beträgt. Überraschenderweise wurde herausgefunden, dass die Absorptionsgeschwindigkeit vorteilhaft ist, wenn die an zu behandelnder Haut adhäsive Hautkontaktschicht Öffnungen mit einer kreisförmigen Form und einem durchschnittlichen Durchmesser von 2,2 mm bis 2,8 mm aufweist.

Obwohl es möglich ist, eine an zu behandelnder Haut adhäsive Hautkontaktschicht bereitzustellen, die im Wesentlichen aus einem weichen Hautklebstoff-Silikonelastomer besteht, ist es bevorzugt, wenn diese Hautkontaktschicht eine zusätzliche Lage eines Öffnungen aufweisenden Materials enthält, deren Öffnungen mit den Öffnungen in der Silikonklebstoffschicht korrespondieren, ohne diese zu verschließen. Das mit Öffnungen versehene Material kann jede Art von medizinisch akzeptablem Material umfassen, inklusive textile Materialien wie Vliese, Gestricke, Gewirke oder Gewebe. Geeigneter Weise handelt es sich bei dem Öffnungen aufweisenden Material um ein einheitliches Material wie ein Polymernetz oder ein perforierter Film. Geeignete Polymermaterialien umfassen Polyethylen, Polypropylen, Polyester, Polyvinylacetat, Ethylenvinylacetat und Polyurethan. Geeigneter Weise hat das Lagenmaterial eine Dicke von 1 μm bis 100 μm, bevorzugt 5 μm bis 50μm.

Die an zu behandelnder Haut adhäsive Hautkontaktschicht kann auf ihrer distalen Seite mit einer Schicht eines medizinisch akzeptablen Klebstoffs versehen sein. Eine derartige Klebstoffschicht sorgt für eine besonders stabile Verbindung zwischen der an zu behandelnder Haut adhäsiven Hautkontaktschicht und den auf der distalen Seite der an zu behandelnder Haut adhäsiven Hautkontaktschicht angeordneten Bestandteilen des Artikels, insbesondere der Kompresse und/ oder im Randbereich der äußeren Lage. Bevorzugt handelt es sich bei dem Klebstoff um einen druckempfindlichen Acrylatklebstoff.

Ein bevorzugtes Lagenmaterial für die an zu behandelnder Haut adhäsive Hautkontaktschicht ist ein Polyurethanmaterial mit einer Silikonklebstoffschicht in einer Menge von 150 g/ m$^2$, welches unter der Bezeichnung Acrysil® von der Firma ASC (Frankreich) erhältlich ist.

In einer bevorzugten Ausführungsform weist ein erfindungsgemäßer Artikel eine zusätzliche äußere Lage auf, wobei die äußere Lage ein wasserdampfdurchlässiges und im Wesentlichen flüssigkeitsundurchlässiges Filmmaterial aufweist.

Die zusätzliche äußere Lage stützt und stabilisiert die Kompresse und stellt eine Barriere für Mikroorganismen durch den Artikel dar. Die zusätzliche äußere Lage ist im Wesentlichen flüssigkeitsundurchlässig, aber permeabel für Wasserdampf. Bevorzugt weist die zusätzliche äußere Lage ein Filmmaterial auf. Die zusätzliche äußere Lage weist eine Wasserdampfdurchlässigkeit von 300 bis 30000 g/m$^2$/24h, bevorzugt 1000 bis 15000 g/m$^2$/24h und in einer besonders bevorzugten Ausführungsform 1000 bis 5000 g/m$^2$/24h auf, gemessen nach EN 13726. Die zusätzliche äußere Lage hat eine Dicke von 10 $\mu$m bis 500 $\mu$m, bevorzugt von 15 $\mu$m bis 300 $\mu$m, besonders bevorzugt von 20 $\mu$m bis 100 $\mu$m.

Die zusätzliche äußere Lage kann verschiedene geometrische Formen aufweisen, wie quadratisch, rechteckig, rund, oval, trapezförmig oder polygonal, bevorzugt mit abgerundeten Ecken.

Die zusätzliche äußere Lage weist eine proximale und eine distale Oberfläche auf. Die distale Oberfläche weist bevorzugt eine geringe Oberflächenreibung auf. Durch die Verwendung von Materialien mit einer geringen Oberflächenreibung auf der distalen Oberfläche der äußeren Lage werden Scherkräfte reduziert, die bei Kontakt mit der Oberfläche eines anderen Gegenstands, beispielsweise eines Bettlakens, einer Matratze oder eines Kissens auftreten und die Entstehung oder Verschlechterung von Dekubitalulcera begünstigen können.

Geeignete Materialien für die zusätzliche äußere Lage sind Polyurethane, Polalkoyalkylacrylate, Methylacrylate wie in GB 1280631 offenbart. Geeigneter Weise weist die äußere Lage eine kontinuierliche Schicht eines Polyurethanschaums mit hoher Dichte auf, der überwiegend geschlossene Zellen aufweist. Ein geeignetes Material ist ein 30 $\mu$m dicker Polyurethanfilm, der unter der Bezeichnung 1305 der Firma Coveris erhältlich ist.

Bevorzugt ist die zusätzliche äußere Lage transparent, um eine Betrachtung der Kompresse zu ermöglichen. Dies ist vorteilhaft bei der Beurteilung des Zustandes der von dem Artikel abgedeckten Hautareale. Insbesondere lässt sich so frühzeitig erkennen, ob sich der Zustand der unter dem Artikel befindlichen Hautareale im Verlauf der Behandlung verschlechtert.

In einer bevorzugten Ausführungsform weist die proximale Oberfläche der zusätzlichen äußere Lage eine Beschichtung mit einem druckempfindlichen Klebstoff auf und überragt die distale Oberfläche der Kompresse allseitig, so dass sie einen adhäsiven Randbereich bildet, der die Kompresse allseitig umgibt.

Der Klebstoff befestigt die zusätzliche äußere Lage auf weiteren Schichten des Artikels, insbesondere auf der Kompresse und der an zu behandelnder Haut adhäsiven Hautkontaktschicht. Die zusätzliche äußere Lage kann auf ihrer proximalen Seite kontinuierlich oder diskontinuierlich beziehungsweise partiell mit Klebstoff beschichtet sein. Die zusätzliche äußere Lage kann kontinuierlich mit Klebstoff beschichtet sein, was bedeutet, dass der Klebstoff die gesamte proximale Seite der zusätzlichen äußeren Lage mit Klebstoff bedeckt. In einer weiteren Ausführungsform kann der Klebstoff in Form von Streifen, Punkten oder in verschiedenen Mustern aufgetragen sein. In einer weiteren Ausführungsform umfasst die zusätzliche äußere Lage auf ihrer proximalen Seite eine Klebstoffschicht, die einen klebstofffreien Bereich in der Mitte der zusätzlichen äußeren Lage bereitstellt, um den Kontakt mit der distalen Oberfläche der Kompresse zu reduzieren. So wird die Wasserdampfdurchlässigkeit im klebstofffreien Bereich erhöht. Wenn die Kompresse durch Aufnahme von Feuchtigkeit zu quellen beginnt, verändert sich ihre räumliche Ausdehnung. Dies kann einerseits zur Delaminierung aufgrund von Scherkräften innerhalb des Artikels führen. Bedeutender ist jedoch, dass die auftretenden Scherkräfte Stress auf die durch den Artikel abgedeckte Haut ausüben und so die weitere Entstehung oder Verschlechterung von Dekubitalulcera begünstigen können. Weiterhin hat dies den Vorteil, dass die zur Entstehung von Dekubitalulcera beitragende Hautfeuchtigkeit reduziert werden kann. Ein Artikel, der einen klebstofffreien Bereich zwischen distaler Oberfläche der Kompresse und der proximalen Oberfläche der zusätzlichen äußeren Lage bereitstellt, kann in dieser Hinsicht vorteilhaft sein.

Der Klebstoff ist bevorzugt ein druckempfindlicher Klebstoff eines üblicherweise für medizinische Verbände gebrauchten Klebstofftyps. Geeignete druckempfindliche Klebstoffe können auf Acrylaten, Acrylatestern, Polyvinylethylether, Polyurethan Silikon, deren Derivaten, Mischungen oder Copolymeren basieren wie beispielsweise in GB1280631 beschrieben. Das Flächengewicht der aufgetragenen Klebstoffmenge beträgt geeigneter Weise 10 g/m$^2$ bis 100 g/m$^2$, bevorzugt 15 g/m$^2$ bis 50 g/m$^2$, besonders bevorzugt 20 g/m$^2$ bis 30 g/m$^2$.

Überraschenderweise wurde herausgefunden, dass ein erfindungsgemäßer medizinischer Artikel vorteilhafte Eigenschaften im Absorptionsgeschwindigkeitstest aufweist, der in den Beispielen dieser Anmeldung beschrieben ist.

Überraschenderweise wurde eherausgefunden, dass ein erfindungsgemäßer medizinischer Artikel vorteilhafte Eigenschaften hinsichtlich der Balance zwischen Haftkraft auf Haut und Absorptionsgeschwindigkeit aufweist.

Die zusätzliche äußere Lage, die Kompresse und die an zu behandelnder Haut adhäsive Hautkontaktschicht können verschiedene oder gleiche Ausdehnungen aufweisen.

In einer besonderen Ausführungsform ist die distale Oberfläche der Kompresse von der zusätzlichen äußeren Lage abgedeckt. Die zusätzliche äußere Lage hat eine Ausdehnung, die in beiden Flächenrichtungen größer ist als die Fläche der distalen Oberfläche der Kompresse, während die Kompresse und die an zu behandelnder Haut adhäsive Hautkontaktschicht koextensiv sind. Die äußere Lage bildet daher einen Randbereich, der die Kompresse vollständig umgibt. Dieser Artikeltyp wird auch als Insetyp ("island-type") bezeichnet.

In einer besonderen Ausführungsform ist die distale Oberfläche der Kompresse von der zusätzlichen äußeren Lage abgedeckt. Die zusätzliche äußere Lage hat eine Ausdehnung, die in beiden Flächenrichtungen größer ist als die Fläche der distalen Oberfläche der Kompresse. Die äußere Lage bildet daher einen Randbereich, der die Kompresse allseitig überragt. Die an zu behandelnder Haut adhäsive Hautkontaktschicht hat eine Ausdehnung, die in beiden Flächenrichtungen größer ist als die Fläche der proximalen Oberfläche der Kompresse. Diese Hautkontaktschicht bildet daher einen Randbereich, der die Kompresse allseitig überragt. Dabei ist diese Hautkontaktschicht mit der äußeren Lage koextensiv. Dieser Artikeltyp wird auch als Sandwich-Typ ("sandwich-type") bezeichnet.

Der medizinische Artikel, die zusätzliche äußere Lage, der Kern der Kompresse, die Kompresse, die Hülle und die an zu behandelnder Haut adhäsive Hautkontaktschicht können jeweils jedwede geeignete Größe und geometrische Form aufweisen, insbesondere Quadrate, Rechtecke, Kreise, Ovale, Polygone. Falls die geometrische Form irgendeiner dieser Lagen Ecken aufweist, können diese Ecken bevorzugt abgerundet sein. Dies reduziert das Risiko eines Aufrollens des Artikels während der Anwendung und der Delaminierung von weitern Lagen oder der Haut. In einer bevorzugten Ausführungsform ist der Radius für abgerundete Ecken 5 mm bis 15 mm, bevorzugte 12,5 mm.

Ein medizinischer Artikel gemäß der vorliegenden Erfindung kann wenigstens eine Abdeckschicht aufweisen, welche die adhäsiven Bereiche auf der proximalen Oberfläche der an zu behandelnder Haut adhäsiven Hautkontaktschicht bedecken. Die Abdeckschicht bedeckt und schützt die Kompresse und verhindert verfrühtes Anhaften der adhäsiven Teile des Artikels. Eine Abdeckschicht kann einen Film aus Polyethylen, Polypropylen, Fluorkohlenwasserstoffen umfassen sowie Papier, welches mit einem der genannten Materialien oder Silikon beschichtet ist. Ein geeignetes Material kann eine Polyethylenfolie mit einer Dicke von 100 $\mu$m sein, welche von der Firma Flextrus® erhältlich ist.

Ein medizinischer Artikel gemäß der vorliegenden Erfindung kann auf einer Hautpartie eines Patienten aufgebracht und mittels der in dem Artikel enthaltenen an zu behandelnder Haut adhäsiven Hautkontaktschicht fixiert werden und sichert so die Hautpartie gegenüber dem Kontakt mit äußeren Materialien, beispielsweise einem Bettlaken. So wird das Auftreten von Scherkräften vermieden, die normalerweise durch Reibung zwischen der Haut des Patienten und einem äußeren Material auftreten. Da ein medizinischer Artikel gemäß der vorliegenden Erfindung eine gewisse Steifigkeit aufweist, wird die zwischen der äußeren Lage des Artikels und einem äußeren Material auftretende Reibungskraft nicht durch die Schichten des Artikels an die vom Artikel abgedeckte Hautoberfläche übertragen. Somit sind Artikel gemäß der vorliegenden Erfindung in der Lage, das Auftreten von Scherkräften an gefährdeten Hautpartien zu verringern oder sogar zu vermeiden. Die auftretenden Reibungskräfte zwischen äußerer Lage des Artikels und einem äußeren Material können durch Bestimmung des Reibungskoeffizienten abgeschätzt werden. Man unterscheidet zwischen statischem Reibungskoeffizienten, der die Haftreibung zwischen zwei Materialien charakterisiert, und dynamischem Reibungskoeffizienten, der die Gleitreibung zwischen zwei Materialien charakterisiert. Wichtig ist, dass sowohl der statische als auch der dynamische Reibungskoeffizient einen bestimmten Wert nicht übersteigt, um das Risiko für das Auftreten von Scherkräften im von einem Artikel abgedeckten Hautbereich zu minimieren. Reibungskoeffizienten sind abhängig von der Feuchtigkeit. Eine durch Schweiß oder Exsudat befeuchtete Hautoberfläche weist eine höhere Empfindlichkeit für das Auftreten schädlicher Scherkräfte auf als eine trockene Hautoberfläche. Ebenso ist für die Eignung eines Artikels entscheidend, ob die äußere Oberfläche des Artikels seine reibungsverringernden Eigenschaften auch bei erhöhter Feuchtigkeit beibehält. Überraschenderweise wurde herausgefunden, dass sowohl ein statischer als auch ein dynamischer Reibungskoeffizient von 0,20 bis 0,50, bevorzugt von 0,25 bis 0,45 vorteilhaft ist. Statische und dynamische Reibungskoeffizienten können nach einem Verfahren ermittelt werden, das in Beispiel 9 der vorliegenden Anmeldung beschrieben ist.

Ein medizinischer Artikel gemäß der vorliegenden Erfindung kann auch dazu beitragen, den auf eine bestimmte Hautpartie und das darunter liegende Gewebe ausgeübten Druck zu verringern. Druck entsteht, indem eine Kraft auf eine bestimmte Fläche einwirkt, beispielsweise die Gewichtskraft eines Körperteils auf die Hautoberfläche, die in Kontakt mit einem unter dem Körperteil befindliches äußeres Material steht, z. B einem Bettlaken. Damit ein medizinischer Artikel eine Verringerung des Drucks bewirken kann, muss die Kraft, die auf eine Hautpartie wirken kann, vom Artikel aufgenommen und auf eine größere Fläche verteilt werden.

Dazu muss das Artikelmaterial eine bestimmte Kompressibilität aufweisen. Materialien, die zu stark kompressibel sind, leiten den auf die Oberfläche wirkenden Druck unmittelbar in Druckrichtung weiter, ohne für eine signifikante Verteilung der Kräfte auf eine größere Fläche zu sorgen. Dagegen können Materialien, die sehr wenig kompressibel sind, nur wenig Druck aufnehmen. Die Kompressibilität eines erfindungsgemäßen Artikels kann in einem Verfahren bestimmt werden, welches in Beispiel 8 der vorliegenden Anmeldung beschrieben ist. Die Kompressibilität ist definiert durch das Ausmaß der Stauchung des Artikels bei einer bestimmten Kraft. Sie ist ebenfalls von der Feuchtigkeit abhängig. Überraschenderweise wurde herausgefunden, dass ein Artikel, der in trockenem Zustand bei einer Kraft von 150 N eine Stauchung von 2,0 bis 5,0 mm, bevorzugt 3,0 bis 4,5 mm, besonders bevorzugt 3,5 bis 4,0 mm aufweist, während er in angefeuchtetem Zustand bei einer Kraft von 150 N eine Stauchung von 2,0 bis 6,0 mm, bevorzugt 3,0 bis 5,0 mm, besonders bevorzugt 4,0 bis 4,5 mm aufweist, vorteilhaft für die Vorbeugung der Entstehung oder Verschlechterung von Dekubitalulcera ist.

Ein medizinischer Artikel trägt weiterhin zur Verringerung der auftretenden Scherkräfte bei, wenn er in unterschiedlichen Ausdehnungsrichtungen unterschiedliche Elastizitätsmodule aufweist. Der Elastizitätsmodul ist ein Materialkennwert aus der Werkstofftechnik, der bei linear-elastischem Verhalten den proportionalen Zusammenhang zwischen mechanischer Spannung und Dehnung bei der Verformung eines festen Körpers beschreibt. Mechanische Spannung ist ein Maß für die innere Beanspruchung eines Körpers infolge dessen Belastung von außen, beispielsweise durch Druck und/oder Scherkräfte. Eine Materialschicht, die in einer ersten Richtung einen größeren Elastizitätsmodul aufweist als in einer zweiten dazu rechtwinkligen Richtung, kann sich einer parallel zur ersten Richtung wirkenden Kraft mit einer gewissen Stabilität entgegenstellen und in der dazu rechtwinkligen Richtung nachgeben. Ein Artikel, der in einer zu einer äußeren, Druck ausübenden Kraft oder einer Scherkraft wirkenden parallelen Richtung einen höheren Elastizitätsmodul aufweist als in der dazu rechtwinkligen Richtung, kann dazu beitragen, dass die auftretenden Kräfte aus ihrer ursprünglichen Kraftwirkung von der durch den Artikel abgedeckten Haut und dem darunter liegenden Gewebe abgelenkt werden, so dass die Entstehung oder Verschlechterung von Dekubitalulcera verringert werden kann. Dabei ist vor allem das Verhältnis der Elastizitätsmodule in unterschiedlichen Richtungen, also das Ausmaß der Anisotropie entscheidend. Der Elastizitätsmodul kann in einem zyklischen Zugversuch bestimmt werden, der in Beispiel 7 der vorliegenden Anmeldung beschrieben ist. Überraschenderweise wurde festgestellt, dass eine Anisotropie von 1,5 bis 20,0, bevorzugt von 2,0 bis 10,0, besonders bevorzugt von 2,5 bis 8,0 vorteilhaft für die Vorbeugung der Entstehung oder Verschlechterung von Dekubitalulcera ist.

In einer bevorzugten Ausführungsform weist ein erfindungsgemäßer medizinischer Artikel zwei oder mehr Lagen auf, die eine derartige Anisotropie aufweisen. Dabei weist jede der Lagen jeweils eine Längsrichtung mit einem ersten Elastizitätsmodul und eine zur Längsrichtung parallelen Querrichtung mit einem zweiten Elastizitätsmodul auf, wobei jeweils der erste Elastizitätsmodul größer ist als der zweite Elastizitätsmodul. In diesem Fall können sich die anisotropen Effekte der einzelnen Lagen in vorteilhafter Weise synergistisch ergänzen, wenn die zwei oder mehr Lagen im medizinischen Artikel derartig orientiert werden, dass die Längsrichtung einer ersten derartig anisotropen Lage und die Längsrichtung einer zweiten derartig anisotropen Lage und gegebenenfalls die Längsrichtungen weiterer derartig anisotroper Lagen zueinander parallel orientiert sind.

In einer weiteren Ausführungsform weist ein erfindungsgemäßer medizinischer Artikel ein Element auf, welches zur Kennzeichnung der Materialrichtung geeignet ist, die einen höheren Elastizitätsmodul aufweist. Ein derartiges Element kann so beschaffen sein, dass es durch visuelle, akustische oder haptische Wahrnehmung von einem Anwender des Artikels erkannt und verstanden wird. Derartige Elemente können an einer beliebigen Stelle des Artikels angebracht sein, solange die Information verständlich ist. Bevorzugt wird ein derartiges Element auf einer der Außenseiten des Artikels angebracht. Elemente, die durch visuelle Wahrnehmung erkannt werden können, umfassen beispielsweise farbliche Markierungen, geometrische Symbole, Schriftzeichen, Schriftzüge oder Kombinationen davon. Elemente, die durch haptische Wahrnehmung erkannt werden können, umfassen beispielsweise Prägungen, Erhebungen oder Vertiefungen eines oder mehrerer der im Artikel verwendeten Materialien, die beispielsweise mit dem Tastsinn erspürt werden können. Eine Kennzeichnung der unterschiedlichen Materialrichtungen kann auch dadurch erzielt werden, dass in verschiedenen Richtungen Materialien mit verschiedenen Oberflächen verwendet werden, deren Oberflächenunterschiede vom Tastsinn erspürt werden können. Besonders bevorzugt ist eine Kennzeichnung in Form eines visuell oder haptisch wahrnehmbaren Pfeils, dessen Pfeilrichtung entlang einer bevorzugten Applikationsrichtung auf der zu behandelnden Körperoberfläche orientiert ist. Besonders bevorzugt weist ein erfindungsgemäßer Artikel eine Kennzeichnung in Form eines Pfeiles auf, der parallel zur Körperlängsachse des zu behandelnden Patienten angebracht wird, wobei die Pfeilspitze in Richtung des Patientenkopfes weist. Für Applikationen an Körperstellen, die sich nicht eindeutig in Relation zur Körperlängsachse des Patienten bestimmen lassen, bietet sich eine Kennzeichnung eines erfindungsgemäßen Artikels in Form eines Pfeils an, dessen Pfeilspitze in die Richtung der am wahrscheinlichsten zu erwartenden Druckeinwirkung weist.

Drucksensitive Matten können Informationen liefern, welche Drücke in einer Matratze unterhalb einer Hautoberfläche auftreten. Aber sie geben keine Informationen über die Druckverhältnisse innerhalb des Gewebes, auf welches sie wirken. Mittels einer Simulation, die eine Finite Elemente Methode (FEM) verwendet, lassen sich die Biomechanismen, die zur Entstehung oder Verschlechterung von Dekubitalulcera führen, simulieren und studieren.

Aufgrund einer FEM-Simulation wurde überraschenderweise herausgefunden, dass medizinische Verbände nach der vorliegenden Erfindung in der Lage sind, die Entstehung oder Verschlechterung von Dekubitalulcera zu verhindern, wenn sie auf Hautstellen appliziert werden, die häufig zur Entstehung von Dekubitalulcera neigen.

Ein vorstehend beschriebener medizinischer Artikel eignet sich daher zur Prophylaxe der Entstehung von Dekubitalulcera. In diesem Fall wird der Artikel auf eine noch intakte Hautstelle aufgebracht, die besonders von der Entstehung von Dekubitalulcera gefährdet ist.

Ein vorstehend beschriebener medizinischer Artikel eignet sich auch zur Anwendung auf Hautstellen, an denen bereits ein Dekubitalulcus entstanden ist. Diese Stellen können sich unter anderem durch offene Hautwunden auszeichnen, die mehr oder weniger stark Wundexsudat abgeben. Bei nicht adäquater Behandlung besteht bei derartigen Dekubitalulcera die Gefahr, dass sich ihr Zustand im weiteren Verlauf verschlechtert. In diesem Fall eignet sich der Artikel zur Prophylaxe der Verschlechterung und zur therapeutischen Behandlung des bereits entstandenen Dekubitalulcus. Da der Artikel sowohl flüssigkeitsabsorbierende als auch Druck und Scherkräfte reduzierende Eigenschaften aufweist, ist ein erfindungsgemäßer Artikel in besonderem Maße für diese Anwendung geeignet.

Die vorliegende Erfindung umfasst ferner einen erfindungsgemäßen Artikel zur Anwendung in der Prophylaxe der Entstehung von Dekubitalulcera.

Ferner umfasst die vorliegende Erfindung einen erfindungsgemäßen Artikel zur Anwendung in der Prophylaxe der Verschlechterung bereits entstandener Dekubitalulcera.

Die vorliegende Erfindung umfasst außerdem einen erfindungsgemäßen Artikel zur Anwendung in der therapeutischen Behandlung eines bereits entstandenen Dekubitalulcus.

Die vorliegende Erfindung umfasst auch die Verwendung eines erfindungsmäßen medizinischen Artikels zur Herstellung eines Medizinproduktes zur Prophylaxe der Entstehung von Dekubitalulcera.

Die vorliegende Erfindung umfasst auch die Verwendung eines erfindungsmäßen medizinischen Artikels zur Herstellung eines Medizinproduktes zur Prophylaxe der Verschlechterung bereits entstandener Dekubitalulcera.

Figur 1 zeigt einen medizinischen Artikel in Draufsicht gemäß einer bevorzugten Ausführungsform der vorliegenden Erfindung.

Figur 2 zeigt den Artikel der Figur 1 in einer Querschnittsansicht entlang der Schnittlinie A-A von Figur 1.

Figur 3 zeigt einen Probekörper gemäß Typ 5A der DIN ISO 527 mit seinen charakteristischen Abmessungen, wie er für zyklische Zugversuche nach Beispiel 7 verwendet wird.

Figur 4 zeigt ein Diagramm, in dem Ergebnisse der FEM-Berechnung gezeigt werden. Die Abszisse zeigt an der Haut auftretende Von Mises-Spannungen. Die Ordinate zeigt den Anteil des relevanten Vergleichsvolumens (Volume of Interest, VOI), welches Von Mises-Spannungen aufweist, die mindestens so groß sind, wie die zugehörigen Abszissenabschnitte.

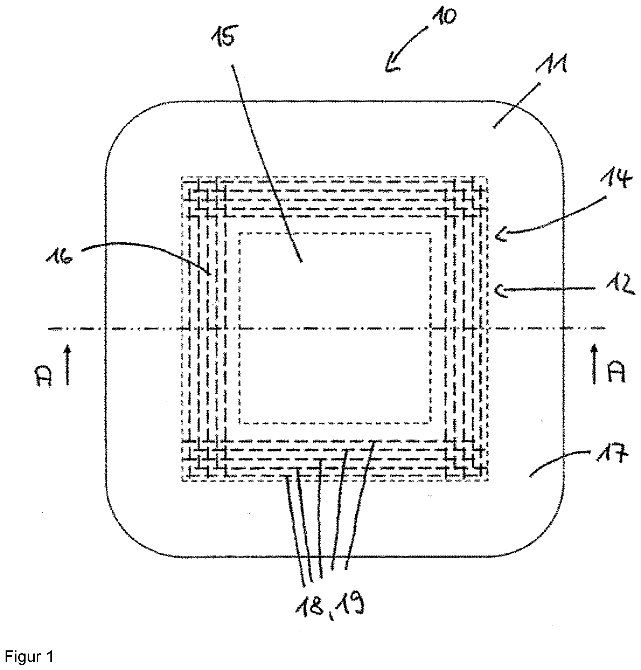

Figur 1 zeigt eine Draufsicht auf einen erfindungsgemäßen medizinischen Artikel (10) mit einer zusätzlichen äußeren Lage (11) und einer Kompresse (12). Eine an zu behandelnder Haut adhäsive Hautkontaktschicht ist auf der vom Betrachter abgewandten Seite angebracht. Die Kompresse (12) umfasst einen Kern (15) und eine Hülle (14), die den Kern (15) umgibt. Die Hülle (14) umfasst eine erste proximale Lage (vom Betrachter abgewandt) eines flüssigkeitsdurchlässigen Vliesmaterials und eine zweite distale Lage (14b) eines im Wesentlichen flüssigkeitsundurchlässigen Vliesmaterials. Die proximale Lage der Hülle (14) bedeckt die proximale Seite des Kerns (15) und überragt die proximale Seite des Kerns (15), wobei ein Randbereich (16) gebildet wird, der den Kern (15) allseitig umgibt. Die distale Lage (14b) der Hülle (14) bedeckt die distale Seite des Kerns (15) und überragt die distale Seite des Kerns, wobei ein Randbereich (16) gebildet wird, der den Kern (15) allseitig umgibt. Die proximale Lage der Hülle (14) und die distale Lage (14b) der Hülle (14) sind entlang des Randbereichs (16), der den Kern (15) umgibt, mittels eines thermischen Prozesses miteinander verbunden worden und besitzen daher eine Schweißverbindung (18). Die Schweißverbindung (18) wird durch fünf diskontinuierliche Schweißlinien (19) gebildet. Die äußere Lage (11) überragt die Kompresse (12), wobei ein Randbereich (17) gebildet wird, der die Kompresse (12) allseitig umgibt.

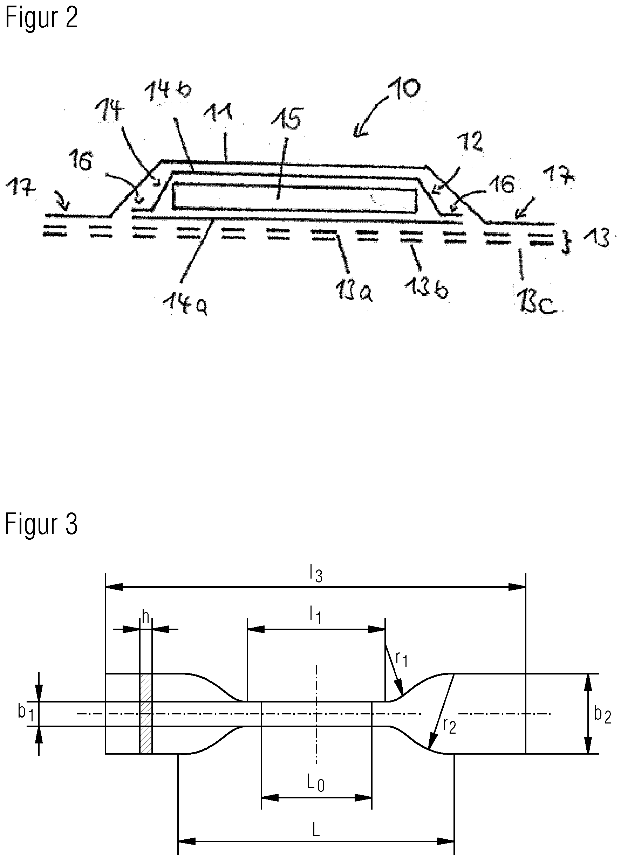

Figur 2 zeigt eine Querschnittansicht des erfindungsgemäßen medizinischen Artikels (10) der Figur 1 entlang der Schnittline A-A aus Figur 1 mit einer Kompresse (12), einer zusätzlichen äußeren Lage (11) und einer Hautkontaktschicht (13). Die Kompresse (12) umfasst einen Kern (15) und eine Hülle (14). Die Hülle (14) wird gebildet aus einer proximalen Lage (14a) und einer distalen Lage (14b). Die Hautkontaktschicht (13) wird gebildet aus einer Lage aus einem perforierten Flächenmaterial (13a) und einer Lage eines hautfreundlichen Silikonklebstoffs (13b). Die äußere Lage (11) wird gebildet aus einem wasserdampfdurchlässigen und flüssigkeitsundurchlässigen Polyurethanfilmmaterial mit geringen Reibungseigenschaften mit einer Dicke von 30 $\mu$m. Die äußere Lage (11) ist mit einer Schicht eines Acrylat-basierten Klebstoffs beschichtet (nicht gezeigt). Der Kern (15) umfasst eine Mischung aus Cellulosefasern und superabsorbierenden NatriumPolyacrylatpartikeln in einem vorgefertigten Airlaid-Material. Der Kern (15) umfasst außerdem eine Diffusionsschicht (nicht gezeigt) in Form einer Lage eines Cellulosepapiers, welches um die Vlieslage aus einer Mischung aus Cellulosefasern und Polyacrylatpartikeln herumgelegt ist. Die proximale Lage (14a) der Hülle (14) ist ein Vliesmaterial aus einer Mischung aus Viskosefasern und Polyamidfasern. Die distale Lage (14b) der Hülle (14) ist ein Vliesmaterial aus Polypropylenfasern. Die proximale Lage (14a) der Hülle (14) bedeckt die proximale Seite des Kerns (15) und überragt die proximale Seite des Kerns (15), wobei sie einen Randberiech (16) bildet, der den Kern (15) allseitig umgibt. Die distale Lage (14b) der Hülle (14) bedeckt die distale Seite des Kerns (15) und überragt die distale Seite des Kerns (15), wobei ein Randbereich (16) gebildet wird, der die Kompresse (12) allseitig umgibt. Die proximale Lage (14a) der Hülle (14) und die distale Lage (14b) der Hülle (14) sind entlang ihres Randbereichs (16), der den Kern (15) umgibt, miteinander verbunden. Das perforierte Flächenmaterial (13a) der Hautkontaktschicht (13) besteht aus einem Polyurethanfilm, welcher Öffnungen (13c) mit einer Kreisform aufweist. Diese Öffnungen (13c) haben eine einheitliche Form mit einem mittleren Durchmesser von 2,4 mm und sind in einem regelmäßigen Muster angeordnet resultierend in einem Anteil der offenen Fläche von 15 %. Die äußere Lage (11) überragt die Kompresse (12). Die Hautkontaktschicht (13) überragt die Kompresse (12), wobei ein Randbereich (17) gebildet wird, der die Kompresse (12) allseitig umgibt. Die äußere Lage (11) und die Hautkontaktschicht (13) sind koextensiv.

Figur 3 zeigt einen Probenkörper, wie er zur Bestimmung der Materialeigenschaften in den Versuchen gemäß Beispiel 7 (Zugfestigkeit) verwendet wird mit den jeweiligen Dimensionsangaben.

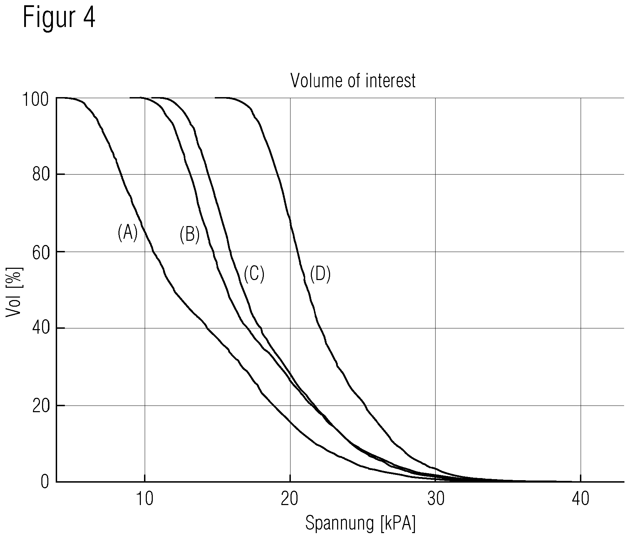

Figur 4 zeigt eine Grafik der im relevanten Modellvolumen (Volume of interest, VOI) auftretenden Spannungen (gemessen in kPa). Dabei gibt die Ordinate an, welcher Anteil des relevanten Volumens eine Spannung aufweist, die mindestens dem zum jeweiligen Kurvenpunkt zugehörenden Wert auf der Abszisse entspricht. Kurve (A) zeigt die Spannungen an, die bei Applikation eines erfindungsgemäßen Gegenstands gemäß Beispiel 1 an der Haut im relevanten Vergleichsvolumen auftreten. Kurve (D) zeigt die Spannungen an, die im relevanten Vergleichsvolumen ohne weitere protektive Maßnahmen auftreten. Die Kurven (B) und (C) zeigen die Spannungen an, die im relevanten Vergleichsvolumen unter Einsatz zweier verschiedener Wettbewerbsprodukte auftreten.

Beispiele

Beispiel 1: Medizinischer Artikel

Der medizinische Artikel umfasst eine Kompresse, eine zusätzliche äußere Lage und eine an zu behandelnder Haut adhäsive Hautkontaktschicht. Die Kompresse umfasst einen Kern und eine Hülle. Die Hülle wird von einer proximalen Lage und einer distalen Lage gebildet. Die Hautkontaktschicht wird aus einem perforierten Flächenmaterial und einer Lage eines hautfreundlichen Silikonklebstoffs gebildet. Die äußere Lage wird aus einem wasserdampfdurchlässigen und flüssigkeitsundurchlässigen Polyurethan-Filmmaterial mit geringen Reibungseigenschaften und einer Dicke von 30 $\mu$m gebildet. Der Kern umfasst eine Mischung aus Cellulosefasern und supersabsorbierenden Natriumpolyacrylatpartikeln in einem vorgefertigten Airlaid-Material. Der Kern umfasst außerdem eine Diffusionsschicht in Form einer Lage eines Cellulosepapiers, welches um die luftgelegte Mischung aus Cellulosefasern und Polyacrylatpartikeln herumgelegt worden ist. Die proximale Lage der Hülle ist ein Vliesmaterial aus einer Mischung aus Viskosefasern und Polyamidfasern. Die distale Lage der Hülle ist ein Vliesmaterial aus Polypropylenfasern. Das perforierte Flächenmaterial der Hautkontaktschicht besteht aus einem Polyurethanfilm, der Öffnungen mit kreisrunder Form aufweist. Diese Öffnungen haben eine einheitliche Form mit einem mittleren Durchmesser von 2,4 mm und sind in einem regelmäßigen Muster angeordnet, so dass der Anteil der offenen Fläche 15 % beträgt. Die Hautkontaktschicht umfasst zusätzlich eine Lage eines hautfreundlichen Silikonklebers, ohne dabei die Öffnungen des perforierten Flächenmaterials zu verschließen.

Beispiel 2: Testlösungen, die zur Charakterisierung medizinischer Verbände benutzt werden

Lösung A (Salzlösung)

2 L entionisiertes Wasser
0,74 g Kalziumchloriddihydrat (CaCl$_2$ •2 H$_2$O, CAS: 10035-04-5)
16,6 g Natriumchlorid (NaCl, CAS: 7647-14-5)

Lösung B (Exsudatlösung)

1 l entionisiertes Wasser
70 g Albumin aus Hühnereiweiß (CAS: 9006-59-1)
0,2 g Allura Rot AC (CAS: 25956-17-6)
9g Natriumchlorid (NaCl, CAS: 7647-14-5)
0,37 g Kalziumchloriddihydrat (CaCl$_2$ •2 H$_2$O, CAS: 10035-04-5)
2 g Methyl-4-hydroxybenzoat (CAS: 99-76-3)
1 g Propyl-4-hydroxybenzoat (CAS: 94-13-3)

Beispiel 3: Absorptionsgeschwindigkeit

Die Absorptionsgeschwindigkeit eines Artikels wird bestimmt durch die Zeit, die benötigt wird, um eine Testflüssigkeit vollständig zu absorbieren. Testlösungen können entweder Salzlösung (Lösung A) oder Exsudatlösung (Lösung B) sein. Sowohl die Lösungen als auch die Probenköroper müssen bei Raumtemperatur vor dem Test vorkonditioniert werden, indem die Probenkörper und die Lösungen für zwei Stunden bei 22 °C aufbewahrt werden.

Eine 50 mL Bürette wird mit Testlösung befüllt. Der Flüssigkeitsstand wird bei 15 mL festgelegt. Ein Probenkörper wird unter der Bürette platziert, wobei die Oberfläche der Bürette entgegenzeigt, die bei Verwendung des Artikels der Haut zugewandt ist. Der Abstand zwischen Bürette und Probenkörper wird auf 1 cm festgesetzt. Der Bürettenhahn wird geöffnet. während gleichzeitig eine Stoppuhr gestartet wird. Dabei werden 2 mL der Testlösung aus der Bürette auslaufen gelassen. Die Stoppuhr wird gestoppt, sobald 2 mL der Testlösung vollständig von dem Artikel absorbiert worden sind, d.h. wenn kein Tropfen der Testlösung mehr auf der Perforation der Hautkontaktschicht verbleibt. Falls der Probenkörper groß genug ist, können 1 bis 3 Messungen auf demselben Artikel durchgeführt werden. Die Testpositionen sind dabei in der Mitte sowie zwei weitere Punkte entlang einer Diagonalen in Richtung der Ecken des Artikels, entsprechen den Positionen der Punkte der Zahl 3 auf einem üblichen Spielwürfel.

Wenigstens fünf Proben werden untersucht.

Jeder Wert wird in folgender Weise klassifiziert:

| Time (s) | [0 to 4,9] | [5,0 to10,9] | [11,0 to 30,9] | [31,0 to 60,0] | >60 |
|---|---|---|---|---|---|
| Kategorie | sofort | Sehr schnell | schnell | durchschnittlich | langsam |

Ein Artikel gemäß der vorliegenden Erfindung wurde mit kommerziell erhältlichen Verbänden Biatain[®] silicone, Allevyn[®] Life, Mepilex[®] border verglichen.

Absorptionsgeschwindigkeit

| Artikel | Absorptionsgeschwindigkeit [Anteil der Proben] | | | | |
|---|---|---|---|---|---|
| | sofort | Sehr schnell | schnell | durchschnittlich | langsam |
| Beispiel 1 | 20 % | 80 % | 0 % | 0 % | 0 % |
| Biatain[®] silicone | 0 % | 0 % | 0 % | 20 % | 80 % |
| Allevyn[®] Life | 0% | 0% | 100 % | 0% | 0% |
| Mepilex[®] border | 0% | 0% | 0% | 100 % | 0% |

Beispiel 4: Absorptionskapazität

Sowohl die verwendeten Lösungen (Salzlösung oder Exsudatlösung) als auch die Prüfkörper müssen bei Raumtemperatur vor dem Testen vorkonditioniert werden, indem sie für zwei Stunden bei einer Temperatur von 22 °C aufbewahrt werden.

Die Grundmasse $m_1$ eines Artikels wird nach Entfernung der die Hautkontaktschicht abdeckenden Schutzschicht bestimmt. Die Länge und Breite des Kerns der Kompresse wird bestimmt, so dass die Oberfläche S des Kerns der Kompresse ermittelt werden kann. Eine Schale wird mit Testflüssigkeit befüllt. Die Masse der Testlösung sollte wenigstens 40 mal größer sein als die des Artikels. Der Artikel wird in die Schüssel getaucht, während gleichzeitig eine Stoppuhr gestartet wird. Die Seite des Artikels, die im Gebrauch der Haut zugewandt ist, sollte dem Boden der Schale zugewandt sein, die Rückseite des Artikels sollte oben liegen. Der Artikel sollte nicht am Boden der Schale anhaften. Der Artikel wird in der Schale für 30 min +/- 1 min belassen. Die Verbände sollten nur an ihren Rändern und nicht am Kern der Kompresse selbst angefasst werden. Die Verbände werden an einer Ecke in einer Klammer fixiert und für 20 min bei Raumtemperatur hängen gelassen. Die feuchte Masse $m_2$ des Artikels wird bestimmt. Die Menge an absorbierter Testlösung $m_{liquid}$ wird folgendermaßen berechnet:

$$m_{liquid} = m_2 - m_1$$

Die Absorptionskapazität ist der Quotient aus der Menge an absorbierter Testlösung geteilt durch die Oberfläche S des Kerns der Kompresse mit der Einheit g/ 100 cm$^2$.

$$\text{Absorptionskapazität} = (m_2 - m_1/S)*100$$

Ein Artikel gemäß der vorliegenden Erfindung wurde mit kommerziell erhältlichen Verbänden Biatain® silicone, Allevyn® Life, Mepilex® border.

Vergleich der Absorptionskapazitäten:

| Artikel | Absorptionskapazität in g/ 100 cm$^2$ |
|---|---|
| Beispiel 1 | 147 |
| Biatain® silicone | 116 |
| Allevyn® Life | 87 |
| Mepilex® border | 54 |

Beispiel 5: Haftungsvermögen

Probanden wurden über den Untersuchungszweck aufgeklärt und gaben ihr schriftliches Einverständnis. Testprodukte wurden auf dem Rücken jedes einzelnen Probanden appliziert (zwei Proben pro Testprodukt): Eine Probe jedes Testprodukts wurde auf der oberen Hälfte des Rückens angebracht. Die zweite Probe wurde auf der unteren Hälfte des Rückens angebracht.

Das untere Ende jeder Probe wurde in einem Abstand von 0,5 cm vom unteren Rand umgefaltet, um einen Anfangspunkt zum Abziehen der Probe von unten nach oben mit einer Universaltestmaschine bereitzustellen. Die Proben wurden durch geschultes Personal appliziert und mit Hilfe einer Metallrolle auf die Haut des Probanden gedrückt (1 kg, fünfmaliges Hin- und Herrollen).

Die Probanden kamen 3 Stunden und 50 Minuten nach Probenapplikation zum Studienort und verblieben für wenigstens 10 Minuten in einem klimatisierten Raum. Es wurde überprüft, dass die Probanden nicht schwitzten. 4 Stunden nach Applikation der Proben wurden die Proben mittels einer Universalprüfmachine zur Bestimmung des Haftungsvermögens abgezogen.

Universalprüfmaschine: Zwick 1120 (Zwick GmbH, Ulm, Deutschland). Die Universalprüfmaschine misst die Kraft, die benötigt wird, um die Proben von der Haut eines Probanden abzulösen. Zum Ablösen der Proben wurden die Probanden in einer sitzenden Position platziert. Die Proben wurden in einem Winkel von annähernd 135 ° abgezogen. Pro Probe wurde eine Messung durchgeführt, von jedem Produkt wurden zwei Proben untersucht.

Beispiel 6: Schweißfestigkeit

Die Bestimmung der Schweißfestigkeit wird mit dem Gerät Tensile Tester MTS C42.503E (MTS Systems Corporation, Eden Prairie, USA) bei einer Zellstärke von 50 N durchgeführt.

Proben wurden vorbereitet durch Ausstanzen eines Teils des Kerns der Kompresse, der eine Schweißlinie enthält, mit einer rechteckigen Form einer Breite von 15 mm und einer Länge von 25 mm, wobei die 15 mm Seite mit der Schweißlinie korrespondiert. Die Probe muss wenigstens 2 mm von der Ecke des Produkts entfernt entnommen werden sein. Nach dem Herausstanzen wurde das Kernmaterial entnommen, so dass lediglich ein Stück aus zwei Vliesmaterialien verbleibt, welche miteinander verbunden sind. Die Klammern des Testgeräts wurden so angelegt, dass der Abstand zwischen ihnen 2 cm betrug. Beide Vliesstücke wurden in getrennten Klammern eingespannt. Das Testgerät wurde mit einer Geschwindigkeit von 200 mm/min gestartet bis die beiden Vliesstücke voneinander getrennt wurden.

Die Schweißfestigkeit ist die mittlere Stärke über die Testperiode. Sie wird in N/ 15 mm angegeben.

Beispiel 7: Zugfestigkeit

Probenentnahme

Aus den untersuchten Produkten werden Probekörper entsprechend Typ 5a gemäß DIN ISO 527 ausgestanzt, deren Form in Figur 3 wiedergegeben ist. Die Abmessungen entsprechen folgenden Werten:

| $l_1$ | Länge des engen parallelen Teils | 25 ± 1 |
|---|---|---|
| $l_3$ | Gesamtlänge | ≥ 75 |
| $b_1$ | Breite des engen Teils | 4 ± 0,1 |
| $b_2$ | Breite an den Enden | 12,5 ± 1 |

(fortgesetzt)

| | | |
|---|---|---|
| $r_1$ | Kleiner Radius | $8 \pm 1$ |
| $r_2$ | Großer Radius | $12{,}5 \pm 1$ |
| L | Anfangsabstand der Klemmen | $50 \pm 2$ |
| $L_0$ | Messlänge | $20 \pm 0{,}5$ |

Probekörper wurden sowohl in einer Richtung entnommen, die parallel zur Maschinenrichtung des Herstellungsverfahrens verläuft als auch in einer Richtung, die rechtwinklig zur Maschinenrichtung des Herstellungsverfahrens verläuft.

Zyklische Zugprüfung

Die gestanzten Proben wurden im zyklischen Zugversuch auf ihre mechanischen Eigenschaften untersucht bei einer Prüftemperatur von 35°C mittels einer Universal-Prüfmaschine der Firma Zwick/Roell des Typs Z005 mit Temperaturkammer. Die Proben wurden zweimal bis zu einer nominellen Dehnung von 15% gedehnt und danach entlastet und anschließend bis Bruch geprüft.

Versuchsdurchführung

| Messgerät: | Zwick/Roell Z005 (max. 5 kN) |
|---|---|
| Kraftmessdose: | 500 N |
| Wegaufnehmer: | Traverse |
| Freie Einspannlänge der Probe: | lo = 50 mm |
| Vorkraft: | 0,1 N (Messung beginnt nach Erreichen der Vorkraft (Kraft = 0; Weg = 0) |
| Prüfgeschwindigkeit bis Erreichen der Vorkraft: | 5 mm/min |
| Prüfgeschwindigkeit: | 50 mm/min |
| Anzahl der Be- und Entlastungszyklen: | 2 |

Ls

| Zyklische Belastung: | |
|---|---|
| bis 15% nominelle Dehnung ( $\varepsilon_{nom} = \frac{\Delta s}{s_0}$ ) | |
| Haltezeit bei 15% nominelle Dehnung: | 1 Sekunde |
| Gemessene Werte: | Kraft F und Traversenweg $\Delta s$ |
| Prüftemperatur: | 35°C |
| Prüfatmosphäre: | Luft |

Herstellung der Probekörper durch Stanzen:

| Probe: | taillierte Zugprobe Typ 5A (DIN EN ISO 527-2:2012-06) |
|---|---|
| Probenbreite: | 4,1 mm |

Dickenmessung

| Messgerät: | Messtaster Typ HEIDENHAIN N221 |

Im Zugversuch wurden die Kräfte und Verformungswege gemessen und als Kraft-Weg-Diagramme für die verschiedenen, untersuchten Proben dargestellt.

Der Elastizitätsmodul ist als Steigung des Graphen im Spannungs-Dehnungs-Diagramm definiert:

$$\text{Elastizitätsmodul } E = \sigma / \varepsilon$$

mit Spannung $\sigma$ und Dehnung $\varepsilon$.

Dabei bezeichnet $\sigma = F / A$ die mechanische Spannung Kraft pro Querschnittsfläche des Probekörpers.

Dabei bezeichnet $\varepsilon = \Delta l / l_0$ mit der Längenänderung $\Delta l = l - l_0$ die Dehnung, wobei $l$ die Länge des Probekörpers nach dem Zugversuch und $l_0$ die Ausgangslänge des Probekörpers bezeichnen.

Es wurden sowohl Probekörper untersucht, die in einer Richtung entnommen wurden, die parallel zur Maschinenrichtung des Herstellungsverfahrens verläuft, als auch Probekörper, die in einer Richtung entnommen wurden, die rechtwinklig zur Maschinenrichtung des Herstellungsverfahrens verläuft. Deren Elastizitätsmodule wurden miteinander ins Verhältnis gesetzt zur Berechnung der Anisotropie der jeweiligen Materialien.

In der folgenden Tabelle sind die auf diese Weise ermittelten Anisotropien hinsichtlich der Elastizitätsmodule der einzelnen Bestandteile eines medizinischen Artikels gemäß Beispiel 1 aufgeführt:

| Lage | Anisotropie |
| --- | --- |
| Äußere Lage | 1,1 |
| Kompresse | 2,5 |
| Vliesmaterial | 3,7 |
| Distale Lage der Hülle | 2,4 |
| Hautkontaktschicht | 0,9 |

Beispiel 8: Kompression

Zyklischer Druckversuch

Die Produkte wurden im zyklischen Druckversuch trocken und mit Wasser benetzt auf ihre mechanischen Eigenschaften untersucht bei einer Prüftemperatur von 35°C mittels einer Universal-Prüfmaschine der Firma Zwick/Roell des Typs Z005 mit Temperaturkammer. Die Proben wurden zweimal bis zu einer Kraft von 150 N gedrückt und danach entlastet und anschließend bis max. 450 N geprüft.

Versuchsdurchführung

| Messgerät: | Zwick/Roell ZOOS (max. 5 kN) |
| Kraftmessdose: | 500 N |
| Wegaufnehmer: | Traverse |
| Vorkraft: | 0,1 N (Messung beginnt nach Erreichen der Vorkraft (Kraft = 0; Weg = 0)) |
| Prüfgeschwindigkeit bis Erreichen der Vorkraft: | 2 mm/min |
| Prüfgeschwindigkeit: | 2 mm/min |
| Anzahl der Be- und Entlastungszyklen: | 2 |
| Zyklische Belastung: | bis 150 N Kraft |
| Haltezeit bei 150 N: | Keine |
| Gemessene Werte: | Kraft F und Traversenweg Ds Abstand der Druckplatten bei Vorkraft (= Anfangsmesslänge) |

(fortgesetzt)

| Druckplatten: | Stahl trocken (Durchmesser = 60 mm) |
| Prüftemperatur: | 35°C |
| Prüfatmosphäre: | Luft |

Herstellung der Probekörper;

| Probe: | Pflaster wurde zentrisch und trocken bzw. nass (befeuchtet mit Wasser) auf untere Druckplatte aufgeklebt |
| Befeuchtung: | Destilliertes Wasser (0,15 mL/ cm$^2$) - eine der Probenfläche entsprechende Wassermenge wurde mittels Pipette auf der hautabgewandten Produktoberfläche aufgebracht und für eine Stunde einziehen lassen, danach erfolgte die Prüfung. |

Beispiel 9: Reibungskoeffizient

Reibungsversuche

Die Proben wurden mit einem Rotationstribometer auf ihr Reibungsverhalten zu einer Baumwolloberfläche im trockenen und befeuchteten Zustand untersucht. Befeuchtet bedeutet, dass die Probe an der Oberfläche auf der Deckschicht mit destilliertem Wasser mit 0,15 ml/ cm$^2$ befeuchtet wurden. Eine der Probenfläche entsprechende Wassermenge wurde mittels Pipette auf die Pflasteroberfläche aufgebracht und für eine Stunde einziehen lassen, danach erfolgte die Prüfung. Dazu wird die Probe auf eine ruhende, ebene Stahlfläche (Durchmesser 60 mm) aufgeklebt. Der rotierende Gegenlaufpartner ist ein Baumwollgewebe das mit doppelseitigem Klebeband auf einen Stahlstempel (Durchmesser 60 mm) aufgeklebt ist. Die Probe und die Gegenlauffläche werden mit einer Kontaktkraft 50 N gegeneinandergepresst, und anschließend wird der Reibungskoeffizient zwischen Probe und rotierender Gegenlauffläche bei unterschiedlichen Gleitgeschwindigkeiten gemessen.

Der Antriebsmotor dreht sich mit unterschiedlichen, definierten Drehzahlen, die Gleitgeschwindigkeit von 1 mm/s bis 100 mm/s entsprechen. Die Gleitgeschwindigkeit wird auf den mittleren Durchmesser von 30 mm berechnet.

Versuchsdurchführung

| Messgerät: | Rotationstribometer Typ Eigenbau |
| Kräfte-Momenten-Sensor: | max.100 N |
| Vorkraft: | 50 N (statisch) entspricht Normalkraft |
| Stempeldurchmesser der Reibkontaktflächen: | 60 mm |
| Gegenlaufpartner: | Baumwolle |
| Gleitgeschwindigkeit v für mittleren Stempeldurchmesser d = 30 mm: | variabel, 1mm/s bis 100 mm/s |
| Drehzahl n: | variabel entsprechende Gleitgeschwindigkeit, $n = \frac{v}{\pi \cdot d} \cdot 60$ |
| Geschwindigkeitsprofil: | 15 Stufen, siehe Tabelle |
| Probenanzahl: | jeweils 3 |
| Prüftemperatur: | 23°C (Raumtemperatur) |
| Prüfatmosphäre: | Luft |

Prüfbedingungen der Reibversuche

| Stufe | Gleitgeschwindigkeit v für Ø 30 mm [mm/s] | Drehzahl n [U/min] | Zeitspanne Δt [s] | Zeit t [s] |
|---|---|---|---|---|
| 1 | 0,0 | 0,00 | 5 | 5 |
| 2 | 0,2 | 0,13 | 5 | 10 |
| 3 | 0,0 | 0,00 | 5 | 15 |
| 4 | 1,0 | 0,64 | 100 | 115 |
| 5 | 5,0 | 3,18 | 20 | 135 |
| 6 | 10 | 6,37 | 10 | 145 |
| 7 | 20 | 12,74 | 10 | 155 |
| 8 | 30 | 19,11 | 10 | 165 |
| 9 | 40 | 25,48 | 10 | 175 |
| 10 | 50 | 31,85 | 10 | 185 |
| 11 | 60 | 38,22 | 10 | 195 |
| 12 | 70 | 44,59 | 10 | 205 |
| 13 | 80 | 50,96 | 10 | 215 |
| 14 | 90 | 57,32 | 10 | 225 |
| 15 | 100 | 63,69 | 10 | 235 |

Die untersuchten medizinischen Verbände wiesen folgende Reibungskoeffizienten (COF) auf:

| | |
|---|---|
| Haftreibung (trocken) | $COF_{trocken} = 0,36$ |
| Haftreibung (angefeuchtet) | $COF_{feucht} = 0,41$ |
| Gleitreibung (trocken) | $COF_{trocken} = 0,28$ |
| Gleitreibung (angefeuchtet) | $COF_{nass} = 0,36$ |

Beispiel 10: Finite Elemente Methode (FEM)

Die FEM ist ein allgemeines und computergestütztes bei unterschiedlichen physikalischen Aufgabenstellungen angewendetes numerisches Verfahren. Logisch basiert die FEM auf dem numerischen Lösen eines komplexen Systems aus Differentialgleichungen. Die FEM teilt große Probleme in eine Vielzahl kleinerer Teile, genannt finite Elemente, auf. Die Analyse wird mit jedem einzelnen dieser Elemente durchgeführt und ergibt in der Gesamtbetrachtung eine Lösung für das gesamte Problem.

Die Arbeitsschritte einer FEM lassen sich wie folgt beschreiben:

1. Kreation eines 2D- oder 3D-Modells, welches aus finiten Elementen besteht;
2. Definition der Materialeigenschaften des Modells;
3. Definition der Randbedingungen und Ladungen zur Anwendung des Modells auf das Problem;
4. Computergestützte Lösung des Problems; und
5. Analyse der Ergebnisse durch Visualisierung und Berechnung.

Die der Erfindung zugrunde liegende FEM-Berechnung erfolgte nach der Methode, die in folgendem Artikel beschrieben ist: Levy A, Schwartz D, Gefen A. The contribution of a directional preference of stiffness to the efficacy of prophylactic sacral dressings in protecting healthy and diabetic tissues from pressure injury: computational modelling studies. Int Wound J 2017; doi: 10.111/iwj.12821

Zum Verständnis der Effekte medizinischer Verbände gemäß der vorliegenden Erfindung wurden FE-Modelle eines menschlichen Beckens und der Verbände geschaffen. Es wurden die Einflüsse von Druck und Stress auf die Haut und tiefere Gewebe analysiert.

Als Grundlage des Beckenmodells dienten MRI-Scans einer freiwilligen Probandin, um möglichst große anatomische Genauigkeit des Modells zu gewährleisten.

Die FE-Modelle umfassen 3.900.000 Knoten.

Weiche Gewebe wurden als nicht-lineare Materialien repräsentiert, wobei Muskeln zu einem Material zusammengefasst wurden und Fett und Haut jeweils als komprimierbare Materialien zusammengefasst wurden. Die Knochen wurden als ein rigider Körper zusammengefasst.

Folgende Materialeigenschaften wurden der Modellierung zugrunde gelegt:

| | |
|---|---|
| Bett: | linearer Elastizitätsmodul E = 50 kPa |
| Knochen: | linearer Elastizitätsmodul E = 7000 MPa |
| Fettgewebe: | Hyperelastizität (Neo-Hooke) C10 = 0,0004 |
| Muskelgewebe: | Hyperelastizität (Neo-Hooke) C10 = 0,000225 |
| Haut: | Hyperelastizität (neo-Hooke) C10 = 0,004 |

Es wurde ein relevantes Modellvolumen (volume of interest, VOI) mit Abmessungen von 6,7 cm × 2,0 cm × 5,1 cm (x-Richtung x y-Richtung x z-Richtung) gebildet, welches Kreuzbein (Os sacrum) und das darumliegende weiche Gewebe inklusive Haut enthält.

Von Mises-Spannung bezeichnet eine fiktive einachsige Spannung, die aufgrund eines bestimmten werkstoffmechanischen bzw. mathematischen Kriteriums eine hypothetisch gleichwertige Materialbeanspruchung darstellt wie ein realer, mehrachsiger Spannungszustand.

Anhand der Von Mises-Spannung kann der wirkliche, im Allgemeinen dreidimensionale Spannungszustand im Bauteil in der Festigkeits- oder in der Fließbedingung mit den Kennwerten aus dem einachsigen Zugversuch (Material-Kennwerte, z. B. Streckgrenze oder Zugfestigkeit) verglichen werden.

Die Von Mises-Spannungen lassen sich nach folgender Formel berechnen:

$$\sigma_{v,M} = \sqrt{\frac{1}{2}\left[(\sigma_I - \sigma_{II})^2 + (\sigma_{II} - \sigma_{III})^2 + (\sigma_{III} - \sigma_I)^2\right]}$$

Dabei sind $\sigma_I$, $\sigma_{II}$, und $\sigma_{III}$ die in den drei Raumrichtungen auftretenden Hauptspannungen.

Von besonderer Bedeutung sind die an der Haut innerhalb des relevanten Modellvolumens auftretenden Spannungen, weil hier sowohl die zur Entstehung und Verschlechterung von Dekubitalulcera verantwortlichen Drücke als auch Scherkräfte auftreten.

Es erfolgte jeweils ein Vergleich zwischen Rechnungen, bei denen ein erfindungsgemäßer medizinischer Artikel auf einer Hautstelle aufgelegt wurde mit derselben Hautstelle ohne angelegten medizinischen Artikel.

Von aussagekräftigem Interesse ist der 10%-Wert. Dieser Wert gibt an, welche maximalen Spannungen in höchstens 10% des relevanten Vergleichsvolumens auftreten. Er entspricht dem Kurvenpunkt, der zu dem Ordinatenabschnitt bei 10% VOI gehört.

Aus dem Vergleich der Von Mises-Spannungen an der Haut im relevanten Modellvolumen mit und ohne angelegtem medizinischen Artikel lässt sich die Eignung zur Vorbeugung der Entstehung von Dekubitalulcera postulieren, sofern der 10%-Wert für die auftretenden Spannungen eine Reduktion von mehr als 10 % erfährt.

Die in Figur 4 gezeigten Kurven weisen folgende 10%-Werte auf:

| | | |
|---|---|---|
| Kurve (A): | 21,8239 kPa | erfindungsgemäßer Artikel |
| Kurve (B): | 24,3311 kPa | Wettbewerbsprodukt 1 |
| Kurve (C): | 24,2884 kPa | Wettbewerbsprodukt 2 |
| Kurve (D): | 27,2725 kPa | ohne aufgelegten Artikel |

Wie aus Figur 4 ersichtlich ist, konnte durch Anwendung eines erfindungsgemäßen medizinischen Artikels der 10%-Wert für die auftretenden Von-Mises-Spannungen an der Haut im relevanten Modellvolumen um 20 % reduziert werden.

**Patentansprüche**

1. Medizinischer Artikel (10) zur Prophylaxe der Entstehung und/oder Verschlechterung von Dekubitalulcera enthaltend eine Kompresse (12) und eine an zu behandelnder Haut adhäsive Hautkontaktschicht (13), wobei die Kompresse (12) eine proximale Oberfläche und eine distale Oberfläche aufweist und einen Kern (15) und eine den Kern (15) umgebende Hülle (14) umfasst, wobei der Kern (15) eine proximale Oberfläche und eine distale Oberfläche aufweist und ein Vliesmaterial aus Fasern und absorbierenden Partikeln umfasst, wobei die Hülle (14) eine erste Lage aus einem flüssigkeitsdurchlässigen Material mit hydrophilen Eigenschaften umfasst, die auf der proximalen Oberfläche des Kerns (15) angeordnet ist, und eine zweite Lage aus einem von dem Material der ersten Lage der Hülle (14) verschiedenen Material, die auf der distalen Oberfläche des Kerns (15) angeordnet ist, umfasst, wobei die erste Lage der Hülle (14) die proximale Oberfläche des Kerns (15) allseitig überragt und die zweite Lage der Hülle (14) ein zweites Material mit hydrophoben Eigenschaften umfasst und die distale Oberfläche des Kerns (15) allseitig überragt, und wobei die erste Lage der Hülle (14) und die zweite Lage der Hülle (14) jeweils einen Randbereich (16) bilden, der den Kern (15) allseitig umgibt, wobei die erste Lage der Hülle (14) und die zweite Lage der Hülle (14) in diesem Randbereich (16) miteinander verbunden sind und wobei die Kompresse (12) eine Längsrichtung mit einem ersten Elastizitätsmodul und eine Querrichtung mit einem zweiten Elastizitätsmodul aufweist und der erste Elastizitätsmodul größer ist als der zweite Elastizitätsmodul und wobei das Verhältnis der Elastizitätsmodule 1,5 bis 20 beträgt.

2. Medizinischer Artikel (10) gemäß Anspruch 1, **dadurch gekennzeichnet, dass** der Kern (15) eine Absorptionskapazität von 130 g/ 100 cm$^2$ bis 3000 g/ 100 cm$^2$, gemessen nach der Testmethode von Beispiel 4 aufweist.

3. Medizinischer Artikel (10) nach einem der Ansprüche 1 bis 2, **dadurch gekennzeichnet, dass** sowohl die erste Lage als auch die zweite Lage der Hülle (14) jeweils ein Material mit thermoplastischen Eigenschaften aufweist.

4. Medizinischer Artikel (10) nach Anspruch 3, **dadurch gekennzeichnet, dass** die zweite Lage der Hülle (14) ein thermoplastisches Material mit hydrophoben Eigenschaften umfasst und die erste Lage der Hülle (14) ein thermoplastisches Material umfasst, welches das gleiche Material wie das der zweiten Lage der Hülle (14) ist und in einem chemischen und/oder physikalischen Verfahren so behandelt wurde, dass es hydrophile Eigenschaften aufweist.

5. Medizinischer Artikel (10) nach einem der Ansprüche 3 bis 4, **dadurch gekennzeichnet, dass** die erste Lage der Hülle (14) und die zweite Lage der Hülle in einem thermischen Prozess miteinander verbunden worden sind und daher eine Schweißverbindung (18) miteinander aufweisen.

6. Medizinischer Artikel (10) nach Anspruch 5, **dadurch gekennzeichnet, dass** die Schweißverbindung (18) wenigstens eine diskontinuierliche Schweißlinie (19) umfasst.

7. Medizinischer Artikel (10) nach Anspruch 6, **dadurch gekennzeichnet, dass** die Schweißverbindung (18) vier bis sechs parallele diskontinuierliche Schweißlinien (19) umfasst.

8. Medizinischer Artikel (10) nach einem der Ansprüche 6 oder 7, **dadurch gekennzeichnet, dass** die wenigstens eine diskontinuierliche Schweißlinie (19) parallel zur Maschinenrichtung im Herstellungsprozess ausgerichtet ist.

9. Medizinischer Artikel (10) gemäß einem der vorgenannten Ansprüche, **dadurch gekennzeichnet, dass** die an zu behandelnder Haut adhäsive Hautkontaktschicht (13) eine Lage eines hautfreundlichen Silikonklebstoffs (13b) aufweist.

10. Medizinischer Artikel (10) nach einem der vorgenannten Ansprüche, **dadurch gekennzeichnet, dass** die Hautkontaktschicht (13) Öffnungen aufweist, deren gesamte offene Fläche zwischen 10 % und 25 % der gesamten Oberfläche der Hautkontaktschicht (13) beträgt.

11. Medizinischer Artikel (10) nach einem der vorgenannten Ansprüche, **dadurch gekennzeichnet, dass** die Hautkontaktschicht (13) Öffnungen (13c) aufweist mit einer im Wesentlichen kreisförmigen Form, deren mittlerer Durchmesser zwischen 0,2 mm bis 3,0 mm beträgt.

12. Medizinischer Artikel (10) gemäß einem der vorgenannten Ansprüche, **dadurch gekennzeichnet, dass** der Artikel eine zusätzliche äußere Lage (11) mit einer proximalen und einer distalen Oberfläche aufweist, wobei die zusätzliche äußere Lage (11) ein wasserdampfdurchlässiges und im Wesentlichen flüssigkeitsundurchlässiges Filmmaterial aufweist.

13. Medizinischer Artikel (10) nach einem der vorgenannten Ansprüche, **dadurch gekennzeichnet, dass** die proximale Oberfläche der zusätzlichen äußeren Lage (11) eine Beschichtung mit einem druckempfindlichen Klebstoff aufweist und die zusätzliche äußere Lage (11) die distale Oberfläche der Kompresse (12) allseitig überragt, so dass sie einen adhäsiven Randbereich (17) bildet, der die Kompresse (12) allseitig umgibt.

14. Medizinischer Artikel (10) nach einem der Ansprüche 1 bis 13, **dadurch gekennzeichnet, dass** die zusätzliche äußere Lage (11) die distale Oberfläche der Kompresse (12) allseitig überragt und die Hautkontaktschicht (13) mit der äußeren Lage (11) koextensiv ist.

15. Medizinischer Artikel (10) nach einem der vorangehenden Ansprüche, **dadurch gekennzeichnet, dass** der Artikel (10) ein Element aufweist, welches zur Kennzeichnung der Materialrichtung geeignet ist, die einen höheren Elastizitätsmodul aufweist.

16. Medizinischer Artikel (10) nach einem der vorangehenden Ansprüche zur Anwendung in der Prophylaxe der Entstehung und/oder Verschlechterung von Dekubitalulcera.


**Claims**

1. Medical article (10) for prophylaxis of the development and/or worsening of decubitus ulcers, containing a compress (12) and a skin-contact layer (13) adhesive to skin to be treated, wherein the compress (12) has a proximal surface and a distal surface and comprises a core (15) and a shell (14) surrounding the core (15), wherein the core (15) has a proximal surface and a distal surface and comprises a nonwoven material composed of fibres and absorbent particles, wherein the shell (14) comprises a first ply composed of a liquid-permeable material having hydrophilic properties and arranged on the proximal surface of the core (15) and comprises a second ply composed of a material different from the material of the first ply of the shell (14) and arranged on the distal surface of the core (15), wherein the first ply of the shell (14) projects beyond the proximal surface of the core (15) on all sides and the second ply of the shell (14) comprises a second material having hydrophobic properties and projects beyond the distal surface of the core (15) on all sides, and wherein the first ply of the shell (14) and the second ply of the shell (14) each form an edge region (16) which surrounds the core (15) on all sides, wherein the first ply of the shell (14) and the second ply of the shell (14) are joined to one another in said edge region (16), and wherein the compress (12) has a longitudinal direction having a first modulus of elasticity and a transverse direction having a second modulus of elasticity and the first modulus of elasticity is greater than the second modulus of elasticity, and wherein the ratio of the moduli of elasticity is 1.5 to 20.

2. Medical article (10) according to Claim 1, **characterized in that** the core (15) has an absorption capacity of from 130 g/100 cm$^2$ to 3000 g/100 cm$^2$, measured in accordance with the test method of example 4.

3. Medical article (10) according to either of Claims 1 to 2, **characterized in that** both the first ply and the second ply of the shell (14) each comprise a material having thermoplastic properties.

4. Medical article (10) according to Claim 3, **characterized in that** the second ply of the shell (14) comprises a thermoplastic material having hydrophobic properties and the first ply of the shell (14) comprises a thermoplastic material which is the same material as that of the second ply of the shell (14) and has been treated in a chemical and/or physical process such that it has hydrophilic properties.

5. Medical article (10) according to either of Claims 3 to 4, **characterized in that** the first ply of the shell (14) and the second ply of the shell have been joined to one another in a thermal process and therefore have a weld connection (18) to one another.

6. Medical article (10) according to Claim 5, **characterized in that** the weld connection (18) comprises at least one discontinuous weld line (19).

7. Medical article (10) according to Claim 6, **characterized in that** the weld connection (18) comprises four to six parallel discontinuous weld lines (19).

8. Medical article (10) according to either of Claims 6 and 7, **characterized in that** the at least one discontinuous weld line (19) is oriented parallel to the machine direction in the production process.

9. Medical article (10) according to any of the preceding claims, **characterized in that** the skin-contact layer (13) adhesive to skin to be treated comprises a ply of a skin-friendly silicone adhesive (13b).

10. Medical article (10) according to any of the preceding claims, **characterized in that** the skin-contact layer (13) has openings, the total open area of which is between 10% and 25% of the total surface area of the skin-contact layer (13).

11. Medical article (10) according to any of the preceding claims, **characterized in that** the skin-contact layer (13) has openings (13c) having a substantially circular shape, the average diameter of which is between 0.2 mm to 3.0 mm.

12. Medical article (10) according to any of the preceding claims, **characterized in that** the article comprises an additional outer ply (11) having a proximal and a distal surface, wherein the additional outer ply (11) comprises a water vapour-permeable and substantially liquid-impermeable film material.

13. Medical article (10) according to any of the preceding claims, **characterized in that** the proximal surface of the additional outer ply (11) comprises a coating containing a pressure-sensitive adhesive and the additional outer ply (11) projects beyond the distal surface of the compress (12) on all sides, so that it forms an adhesive edge region (17) which surrounds the compress (12) on all sides.

14. Medical article (10) according to any of Claims 1 to 13, **characterized in that** the additional outer ply (11) projects beyond the distal surface of the compress (12) on all sides and the skin-contact layer (13) is coextensive with the outer ply (11).

15. Medical article (10) according to any of the preceding claims, **characterized in that** the article (10) comprises an element suitable for marking the material direction having a higher modulus of elasticity.

16. Medical article (10) according to any of the preceding claims for use in the prophylaxis of the development and/or worsening of decubitus ulcers.


**Revendications**

1. Article médical (10) pour la prophylaxie de la naissance et/ou de l'aggravation d'escarres de décubitus contenant une compresse (12) et une couche de contact avec la peau (13), adhérant à la peau à traiter, la compresse (12) présentant une surface proximale et une surface distale et comprenant un noyau (15) et une enveloppe (14) entourant le noyau (15), le noyau (15) présentant une surface proximale et une surface distale et comprenant un matériau non tissé de fibres et de particules absorbantes, l'enveloppe (14) comprenant une première couche en un matériau perméable aux liquides ayant des propriétés hydrophiles, qui est disposé sur la surface proximale du noyau (15), et une deuxième couche d'un matériau différent du matériau de la première couche de l'enveloppe (14), qui est disposé sur la surface distale du noyau (15), la première couche de l'enveloppe (14) dépassant sur tous les côtés de la surface proximale du noyau (15), et la deuxième couche de l'enveloppe (14) comprenant un deuxième matériau ayant des propriétés hydrophiles et dépassant sur tous les côtés de la surface distale du noyau (15), et la première couche de l'enveloppe (14) et la deuxième couche de l'enveloppe (14) formant chacune une zone marginale (16), qui entoure le noyau (15) sur tous les côtés, la première couche de l'enveloppe (14) et la deuxième couche de l'enveloppe (14) étant assemblées l'une à l'autre dans cette zone marginale (16), et la compresse (12) présentant une direction longitudinale ayant un premier module d'élasticité et une direction transversale ayant un deuxième module d'élasticité, et le premier module d'élasticité étant supérieur au deuxième module d'élasticité, et le rapport des modules d'élasticité étant de 1,5 à 20.

2. Article médical (10) selon la revendication 1, **caractérisé en ce que** le noyau (15) présente une capacité d'absorption de 130 g/100 cm$^2$ à 3 000 g/100 cm$^2$, mesurée par la méthode d'essai de l'Exemple 4.

3. Article médical (10) selon l'une des revendications 1 à 2, **caractérisé en ce que** tant la première couche que la deuxième couche de l'enveloppe (14) présentent chacune un matériau ayant des propriétés thermoplastiques.

4. Article médical (10) selon la revendication 3, **caractérisé en ce que** la deuxième couche de l'enveloppe (14) comprend un matériau thermoplastique ayant des propriétés hydrophobes et la première couche de l'enveloppe (14) comprend un matériau thermoplastique qui est le même matériau que celui de la deuxième couche de l'enveloppe (14) et a été traité, dans un procédé chimique et/ou physique, de façon à présenter des propriétés hydrophiles.

**5.** Article médical (10) selon l'une des revendications 3 à 4, **caractérisé en ce que** la première couche de l'enveloppe (14) et la deuxième couche de l'enveloppe ont été assemblées l'une à l'autre par un procédé thermique, et présentent de ce fait une liaison soudée (18) l'une à l'autre.

**6.** Article médical (10) selon la revendication 5, **caractérisé en ce que** la liaison soudée (18) comprend au moins un cordon de soudure discontinu (19).

**7.** Article médical (10) selon la revendication 6, **caractérisé en ce que** la liaison soudée (18) comprend quatre à six cordons de soudure discontinus parallèles (19) .

**8.** Article médical (10) selon l'une des revendications 6 ou 7, **caractérisé en ce que** l'au moins un cordon de soudure discontinu (19) est orienté parallèlement au sens machine dans le procédé de fabrication.

**9.** Article médical (10) selon l'une des revendications précédentes, **caractérisé en ce que** la couche de contact avec la peau (13) adhérant à la peau à traiter présente une couche d'un adhésif siliconé dermophile (13b).

**10.** Article médical (10) selon l'une des revendications précédentes, **caractérisé en ce que** la couche de contact avec la peau (13) présente des ouvertures dont l'aire ouverte totale est comprise entre 10 % et 25 % de l'aire totale de la couche de contact avec la peau (13).

**11.** Article médical (10) selon l'une des revendications précédentes, **caractérisé en ce que** la couche de contact avec la peau (13) présente des ouvertures (13c) ayant pour l'essentiel une forme circulaire dont le diamètre moyen est compris entre 0,2 mm et 3,0 mm.

**12.** Article médical (10) selon l'une des revendications précédentes, **caractérisé en ce que** l'article présente une couche extérieure supplémentaire (11) ayant une surface proximale et une surface distale, la couche extérieure supplémentaire (11) présentant un matériau en film perméable à la vapeur d'eau et pour l'essentiel imperméable aux liquides.

**13.** Article médical (10) selon l'une des revendications précédentes, **caractérisé en ce que** la surface proximale de la couche extérieure supplémentaire (11) présente un revêtement d'un adhésif sensible à la pression, et la couche extérieure supplémentaire (11) dépasse sur tous les côtés de la surface distale de la compresse (12) de façon à former une zone marginale adhésive (17) qui entoure la compresse (12) sur tous les côtés.

**14.** Article médical (10) selon l'une des revendications 1 à 13, **caractérisé en ce que** la couche extérieure supplémentaire (11) dépasse sur tous les côtés de la surface distale de la compresse (12) et la couche de contact avec la peau (13) est coextensive de la couche extérieure (11).

**15.** Article médical (10) selon l'une des revendications précédentes, **caractérisé en ce que** l'article (10) présente un élément qui convient à la caractérisation de la direction du matériau présentant un module d'élasticité plus élevé.

**16.** Article médical (10) selon l'une des revendications précédentes pour une utilisation lors de la prophylaxie de la naissance et/ou de l'aggravation d'escarres de décubitus.

Figur 1

Figur 2

Figur 3

Figur 4

Volume of interest

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 3498243 A1

- GB 1280631 A

### In der Beschreibung aufgeführte Nicht-Patentliteratur

- Standard Test Methods fort he Nonwovens and Related Industries. INDA, Association oft he Nonwoven Fabric Industry, EDANA, International Association Serving the Nonwovens and Related Industries, 2008
- *CHEMICAL ABSTRACTS,* 10035-04-5
- *CHEMICAL ABSTRACTS*, 7647-14-5
- *CHEMICAL ABSTRACTS*, 9006-59-1

- *CHEMICAL ABSTRACTS*, 25956-17-6
- *CHEMICAL ABSTRACTS*, 99-76-3
- *CHEMICAL ABSTRACTS*, 94-13-3
- **LEVY A** ; **SCHWARTZ D** ; **GEFEN A**. The contribution of a directional preference of stiffness to the efficacy of prophylactic sacral dressings in protecting healthy and diabetic tissues from pressure injury: computational modelling studies.. *Int Wound J*, 2017